# EUROPEAN PATENT SPECIFICATION

(11) **EP 1 625 127 B1**
(45) Date of publication and mention of the grant of the patent: **23.05.2007**
(21) Application number: 04731527.0
(22) Date of filing: 06.05.2004
(51) Int. Cl.: C07D 471/04, A61K 31/437, A61P 11/06, A61P 37/00, A61P 19/02, A61P 29/00

(54) **AZAINDOLE COMPOUNDS AS KINASE INHIBITORS**
AZAINDOLVERBINDUNGEN ALS KINASEINHIBITOREN
COMPOSES D'AZAINDOLE EN TANT QU'INHIBITEURS DE KINASE

(30) Priority: 09.05.2003 SE 0301372
(43) Date of publication of application: 15.02.2006
(73) Proprietor: AstraZeneca AB, 151 85 Södertälje (SE)
(72) Inventor: DAVID, Laurent, S-221 87 Lund (SE); HANSEN, Peter, S-221 87 Lund (SE)
(86) International application number: PCT/SE2004/000696
(87) International publication number: WO 2004/099205

(56) References cited:
- WO-A1-20/04016609
- WO-A2-01/47922
- US-A- 5 338 849
- US-A- 5 389 509
- US-B1- 6 686 374

## Description

The present invention relates to novel azaindole compounds which are JAK3 Kinase inhibitors, methods for their preparation, intermediates and pharmaceutical compositions comprising them.

Janus Kinase 3 (JAK3) is a member of the Janus family of protein kinases. Although the other members of this family are expressed by essentially all tissues, JAK3 expression is limited to hematopoetic cells. This is consistent with its essential role in signaling through the receptors for IL-2, IL-4, IL-7, IL-9, IL-13 and IL-15 by non-covalent association of JAK3 with the gamma chain common to these multichain receptors. These cytokines all have a shared function in that they are involved in lymphocyte differentiation and proliferation. XSCID patient populations have been identified with severely reduced levels of JAK3 protein or with genetic defects to the common gamma chain, suggesting that immunosupression should result from blocking signaling through the JAK3 pathway. Animal studies have suggested that JAK3 not only play a critical role in B- and T-lymphocyte maturation, but that JAK3 is constitutively required to maintain T-cell function. Modulation of immune activity through this novel mechanism can prove useful in the treatment of T-cell proliferative disorders such as transplant rejection and autoimmune diseases.

The role of JAK3 in mast cells has been described in knockout mice. Thus, IgE/antigen induced degranulation and mediator release were substantially reduced in mast cells generated from JAK3 deficient mice. JAK3 deficiency does not affect mast cell proliferation in vitro, it has also been shown that IgE receptor levels and mediator contents are identical in JAK3-/- and JAK3 +/+ mast cells. Therefore, JAK3 appears essential for the complete response of IgE challenged mast cells. The role of JAK3 in mast cell activation has been well established in murine system, however, there is no published data on mast cell function in the AR-SCID patients. Targeting JAK3 provides the basis for new and effective treatment of mast cell mediated allergic reactions.

To date a number of JAK3 inhibitors has been disclosed, among them are quinazolines (Sudbeck, E. A. et al. Clinical Cancer Res. 5(1999)1569-82, WO 00/0202) and pyrrolo[2,3-d]pyrimidines (Blumenkopf, T. A. et al. WO 99/65909).

In the current application compounds, 4-anilinoquinoline-3-carboxamides, are claimed as JAK3 inhibitors. Structurally related compounds have previously been described as kinase inhibitors e.g. WO 00/18761 and WO 98/43960 disclose substituted quinoline-3-carbonitrile derivatives. In a recent publication (Boschelli, D.H. et al. J. Med. Chem. 44(2001)822-33) one compound of the present invention has proved not to have any inhibitory capacity towards the activity of the protein tyrosine kinase Src. JAK3 is not mentioned in any of the above literature examples.

WO 02/092571 discloses a series of quinoline derivatives for use in the treatment of a disease mediated by JAK3.

There is a need for further compounds having this activity, and therefore the present invention provides a compound of formula (I): 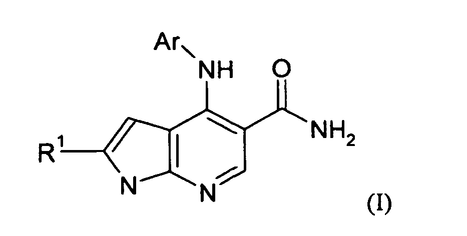 wherein:
R¹ is hydrogen or phenyl optionally substituted by halogen, C₁-C₈ alkoxy, C₁-C₈ thioalkyl or C₁-C₈ alkyl;
Ar is phenyl which can be optionally substituted by one or more groups selected from halogen, hydroxy, cyano, C₁-C₈ alkyl (itself optionally substituted by one or more hydroxy or cyano groups or fluorine atoms), CH₂-R²,; CH₂O(CH₂)ₙOC₁₋₆ alkyl, C₁-C₈ alkyl-NR³-R⁴;
R² is a 5 to 7-membered saturated ring containing 1 or 2 heteroatoms selected from nitrogen, oxygen and sulphur, an aryl or 5- to 7-membered heteroaryl group containing 1 to 3 heteroatoms selected from nitrogen oxygen and suphur, each of which can optionally substituted by one or more substituents selected from hydroxyl or hydroxymethyl;
R³ is hydrogen or C₁₋₆ alkyl and R⁴ is C₁₋₆ alkyl optionally substituted by one or more groups selected from hydroxyl or phenyl,
n is 1 to 4;
and pharmaceutically acceptable salts thereof.

The term alkyl, whether used alone or as part of another group such as alkoxy, means any straight or branched chained alkyl group. The term aryl includes phenyl and naphthyl groups. Compounds of the present invention include all stereoisomers, pure and mixed racemates, and mixtures thereof. Tautomers of compounds of formula (I) also form an aspect of the invention.

Preferably R¹ is hydrogen or phenyl optionally substituted by halogen, in particular fluoro or bromo.

When R² is a 5 to 7-membered saturated ring containing 1 or 2 heteroatoms selected from nitrogen, oxygen and sulphur suitable examples include morpholine, thiomorpholine, azetidine, imidazolidine, pyrrolidine, piperidine and piperazine.

When R² is a 5- to 7-membered heteroaryl group containing 1 to 3 heteroatoms selected from nitrogen oxygen and suphur, examples include thienyl, furanyl, pyrrolyl, imidazolyl, pyridyl, pyrazinyl, pyrimidyl, pyridazinyl, triazinyl, oxazolyl, thiazolyl, isoxazolyl, pyrazolyl, oxadiazolyl, thiadiazolyl, triazolyl, imidazolyl and tetrazolyl.

Preferably Ar is phenyl optionally substituted by one or more CH₂R² , groups where R² is pyrrolidine, morpholine or imidazole each of which is optionally substituted by hydroxyl or hydroxymethyl, or Ar is phenyl optionally substituted by one or more CH₂NR³-R⁴ groups where R³ is hydrogen or methyl and R⁴ is CH₂CH₂OH, CH₂(CH₃)CH₂OH, CH₂(phenyl)CH₂OH, CH₂CH₂(OH)phenyl, CH₂CH₂(OH)CH₂OH, or CH₂OCH₂CH₂OCH₂OH.

Alternatively Ar is phenyl optionally substituted by one or more ethyl or hydroxymethyl groups.

Substituents can be present on any suitable position of the Ar group. More than one substituent can be present, and these can be the same or different. One or two substituent groups are preferred.

Especially preferred compounds of the invention include those exemplified herein, both in free base form and as pharmaceutically acceptable salts.

The invention therefore provides a compound of formula (I) selected from:
4-(2-Ethyl-phenylamino)-2-(4-fluorophenyl)-1*H*-pyrrolo[2,3-*b*]pyridine-5-carboxylic acid amide
4-(2-Ethyl-3-hydroxymethyl-phenylamino)-2-(4-fluorophenyl)-1*H*-pyrrolo[2,3-*b*]pyridine-5-carboxylic acid amide
4-{2-Ethyl-3-[(2-hydroxy-ethylamino)-methyl]-phenylamino}-2-(4-fluoro-phenyl)-1*H-*pyrrolo[2,3-*b*]pyridine-5-carboxylic acid amide
4-(2-Ethyl-3-{[(2-hyroxy-ethyl)-methyl-amino]-methyl}-phenylamino)-2-(4-fluorophenyl)-1*H*-pyrrolo[2,3-*b*]pyridine-5-carboxylic acid amide
4-{2-Ethyl-3-[(2-hydroxy-1-methyl-ethylamino)-methyl]-phenylamino}-2-(4-fluorophenyl)-1*H*-pyrrolo[2,3-*b*]pyridine-5-carboxylic acid amide
4-{ 2-Ethyl-3-[(*S*)-{2-hydroxy-1-phenyl-ethylamino)-methyl]-phenylamino) -2-(4-fluorophenyl)-1*H*-pyrrolo[2,3-*b*]pyridine-5-carboxylic acid amide
4-{2-Ethyl-3-[(2-hydroxy-2-phenyl-ethylamino)-methyl]-phenylamino}-2-(4-fluorophenyl)-1*H*-pyrrolo[2,3-*b*]pyridine-5-carboxylic acid amide
4-(2-Ethyl-3-morpholin-4-ylmethyl-phenylamino)-2-(4-fluoro-phenyl)-1*H*-pyrrolo[2,3-*b*]pyridine-5-carboxylic acid amide
4-[2-Ethyl-3-(3-hydroxy-pyrrolidin-1-ylmethyl)-phenylamino]-2-(4-fluoro-phenyl)-1*H-*pyrrolo[2,3-*b*]pyridine-5-carboxylic acid amide
4-[2-Ethyl-3-((*R*)-2-hydroxymethyl-pyrrolidin-1-ylmethyl)-phenylamino]-2-(4-fluorophenyl)-1*H*-pyrrolo[2,3-*b*]pyridine-5-carboxylic acid amide
4-{3-[(2,3-Dihydroxy-propylamino)-methyl]-2-ethyl-phenylamino}-2-(4-fluoro-phenyl)-1*H*-pyrrolo[2,3-*b*]pyridine-5-carboxylic acid amide
4-(2-Ethyl-3-imidazol-1-ylmethyl-phenylamino)-2-(4-fluoro-phenyl)-1*H*-pyrrolo[2,3-*b*]pyridine-5-carboxylic acid amide
4-[3-(2-Ethoxy-ethoxymethyl)-2-ethyl-phenylamino]-2-(4-fluoro-phenyl)-1*H*-pyrrolo[2,3-*b*]pyridine-5-carboxylic acid amide
2-(4-Bromo-phenyl)-4-(2-ethyl-phenylamino)-1*H*-pyrrolo[2,3-*b*]pyridine-5-carboxylic acid amide
4-(2-Ethyl-phenylamino)-2-phenyl-1*H*-pyrrolo[2,3-*b*]pyridine-5-carboxylic acid amide
4-(2-Ethyl-3-hydroxymethyl-phenylamino)-2-phenyl-1*H*-pyrrolo[2,3-*b*]pyridine-5-carboxylic acid amide
2-(4-Chloro-phenyl)-4-(2-ethyl-3-hydroxymethyl-phenylamino)-1*H*-pyrrolo[2,3-*b*]pyridine-5-carboxylic acid amide
2-(4-Chloro-phenyl)-4-(2-ethyl-3-imidazol-1-ylmethyl-phenylamino)-1*H*-pyrrolo[2,3-*b*]pyridine-5-carboxylic acid amide
4-(2-Ethyl-phenylamino)-1*H*-pyrrolo[2,3-*b*]pyridine-5-carboxylic acid amide,
and pharmaceutically acceptable salts thereof.

Compounds of the invention can form pharmaceutically acceptable solvates and salts. The compounds of the formula (I) can form acid addition salts with acids, such as conventional pharmaceutically acceptable acids, for example maleic, hydrochloric, hydrobromic, phosphoric, acetic, fumaric, salicylic, citric, lactic, mandelic, tartaric, trifluoroacetic and methanesulphonic acids.

The invention also provides a method of treating or preventing a disease mediated by JAK3 which comprises administering to a mammal a compound of formula (I) as defined above.

In a further aspect the invention provides a process for the preparation of a compound of formula (I) which comprises:
reaction of a compound of formula (II): 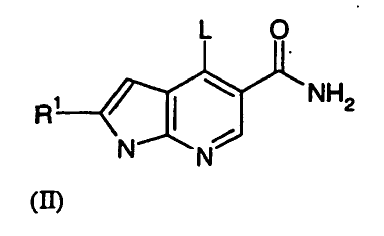
in which R¹ is as defined in formula (I) or is a protected derivative thereof and L is a leaving group, with a compound of formula (III):

Ar-NH₂ (III)

in which Ar is as defined in formula (I) or is a protected derivative thereof, and optionally thereafter:
- removing any protecting groups
- converting a compound of formula (I) into a further compound of formula (I)
- forming a pharmaceutically acceptable salt.

In the above process the group L is a leaving group such as halogen, in particular chloro. The reaction can be carried out in an inert solvent such as NMP at elevated temperature, for example at about 160°C, preferably in a closed vessel.

Compounds of formula (I) can be converted into futher compounds of formula (I) using standard chemistry. For example a compound of formula (I) where Ar is phenyl substituted by a methyl group can be chlorinated using a reagent such as thionyl chloride and the resulting compound treated with a suitable amine to give a further compound of formula (I) as shown below:

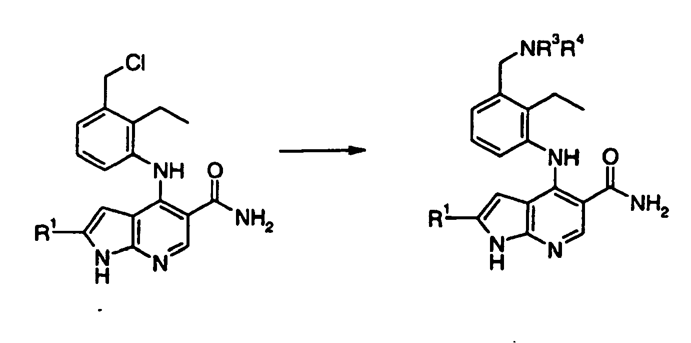

Compounds of formula (II) can be prepared by reacting compounds of formula (VI): 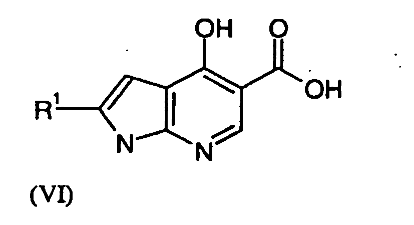 in which R¹ is as defined in formula (II) with a chlorinating agent such as POCl₃ with heating in a closed vessel and reaction of the resulting dichloro compound with aqueous ammonia.

Compounds of formula (VI) can be prepared from compounds of formula (V): 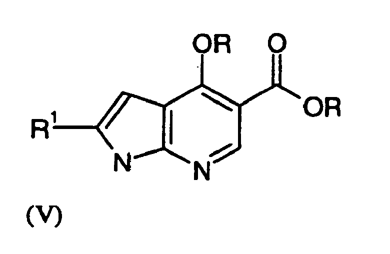 in which R¹ is as defined in formula (II) and the R groups are C₁₋₆alkyl, preferably methyl, by treating with aqueous hydrobromic acid at elevated temperature in a closed vessel.

Compounds of formula (V) can be prepared form compounds of formula (VI): 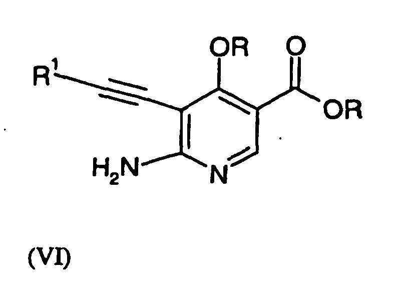 in which R¹ and R are as defined above by treating with a strong base such as KH or KOBu^{t} in a suitable solvent such as dry NMPat ambient or elevated temperature.

Compounds of formula (VI) are prepared using standard chemistry.

It will be appreciated that certain functional groups may need to be protected using standard protecting groups. The protection and deprotection of functional groups is for example, described in 'Protective Groups in Organic Chemistry', edited by J. W. F. McOmie, Plenum Press (1973), and 'Protective Groups in Organic Synthesis', 3rd edition, T. W. Greene & P. G. M. Wuts, Wiley-Interscience (1999).

Diseases mediated by JAK3 include inflammatory, immunological, and bronchopulmonary disorders.

The present invention also relates to a pharmaceutical composition for (a) treating or preventing a disorder or condition selected from organ transplant rejection, lupus, multiple sclerosis, rheumatoid arthritis, psoriasis, Type I diabetes and complications from diabetes, cancer, asthma, rhinitis, atopic dermatitis, autoimmune thyroid disorders, ulcerative colitis, Crohn's disease, Alzheimer's disease, leukemia, and other autoimmune diseases or (b) the inhibition of protein tyrosine kinases or Janus kinase 3 (JAK3) in a mammal, including a human, comprising an amount of a compound of formula I or a pharmaceutically acceptable salt thereof, effective in such disorders or conditions and a pharmaceutically acceptable carrier.

Preferably the compounds of the invention are used for the treatment of asthma, rheumatoid arthritis, and host versus graft rejection/transplantation.

The present invention also relates to a pharmaceutical composition for (a) treating or preventing a disorder or condition selected from organ transplant rejection, lupus, multiple sclerosis, rheumatoid arthritis, psoriasis, Type I diabetes and complications from diabetes, cane, asthma, rhinitis, atopic dermatitis, autoimmune thyroid disorders, ulcerative colitis, Crohn's disease, Alzheimer's disease, leukemia, and other autoimmune diseases or (b) the inhibition of protein tyrosine kinases or Janus kinase 3 (JAK3) in a mammal, including a human, comprising an amount of a compound of formula I or a pharmaceutically acceptable salt thereof, alone or in combination with a T-cell immunosuppresant or anti-inflammatory agents, effective in such disorders or conditions and a pharmaceutically acceptable carrier.

The present invention also relates to a method for the inhibition of protein tyrosine kinases or Janus Kinase 3 (JAK3) in a mammal, including human, comprising administering to said mammal an effective amount of a compound of formula I or a pharmaceutically acceptable salt thereof.

In a still further aspect the invention provides the use of a compound of formula (IA) as a therapeutic agent.

The dose of the compound to be administered will depend on the relevant indication, the age, weight and sex of the patient and may be determined by a physician. The dosage will preferably be in the range of from 0.1 mg/kg to 100 mg/kg.

The compounds may be administered topically, e.g. to the lung and/or the airways, in the form of solutions, suspensions, HFA aerosols or dry powder formulations, e.g. formulations in the inhaler device known as the Turbuhaler^{®}; or systemically, e.g. by oral administration in the form of tablets, pills, capsules, syrups, powders or granules, or by parenteral administration, e.g. in the form of sterile parenteral solutions or suspensions, or by rectal administration, e.g. in the form of suppositories.

The compounds of the invention may be administered on their own or as a pharmaceutical composition comprising the compound of the invention in combination with a pharmaceutically acceptable diluent, adjuvant or carrier. Particularly preferred are compositions not containing material capable of causing an adverse, e.g. an allergic, reaction.

Dry powder formulations and pressurized HFA aerosols of the compounds of the invention may be administered by oral or nasal inhalation. For inhalation the compound is desirably finely divided. The finely divided compound preferably has a mass median diameter of less than 10 µm, and may be suspended in a propellant mixture with the assistance of a dispersant, such as a C₈-C₂₀ fatty acid or salt thereof, (e.g. oleic acid), a bile salt, a phospholipid, an alkyl saccharide, a perfluorinated or polyethoxylated surfactant, or other pharmaceutically acceptable dispersant.

The compounds of the invention may also be administered by means of a dry powder inhaler. The inhaler may be a single or a multi dose inhaler, and may be a breath actuated dry powder inhaler.

One possibility is to mix the finely divided compound with a carrier substance, e.g. a mono-, di- or polysaccharide, a sugar alcohol, or an other polyol. Suitable carriers are sugars, e.g. lactose, glucose, raffinose, melezitose, lactitol, maltitol, trehalose, sucrose, mannitol; and starch. Alternatively the finely divided compound may be coated by another substance. The powder mixture may also be dispensed into hard gelatine capsules, each containing the desired dose of the active compound.

Another possibility is to process the finely divided powder into spheres which break up during the inhalation procedure. This spheronized powder may be filled into the drug reservoir of a multidose inhaler, e.g. that known as the Turbuhaler^{®} in which a dosing unit meters the desired dose which is then inhaled by the patient. With this system the active compound, with or without a carrier substance, is delivered to the patient.

For oral administration the active compound may be admixed with an adjuvant or a carrier, e.g. lactose, saccharose, sorbitol, mannitol; a starch, e.g. potato starch, corn starch or amylopectin; a cellulose derivative; a binder, e.g. gelatine or polyvinylpyrrolidone, and/or a lubricant, e.g. magnesium stearate, calcium stearate, polyethylene glycol, a wax, paraffin, and the like, and then compressed into tablets. If coated tablets are required, the cores, prepared as described above, may be coated with a concentrated sugar solution which may contain e.g. gum arabic, gelatine, talcum, titanium dioxide, and the like. Alternatively, the tablet may be coated with a suitable polymer dissolved in a readily volatile organic solvent.

For the preparation of soft gelatine capsules, the compound may be admixed with e.g. a vegetable oil or polyethylene glycol. Hard gelatine capsules may contain granules of the compound using either the above mentioned excipients for tablets. Also liquid or semisolid formulations of the drug may be filled into hard gelatine capsules.

Liquid preparations for oral application may be in the form of syrups or suspensions, for example solutions containing the compound, the balance being sugar and a mixture of ethanol, water, glycerol and propylene glycol. Optionally such liquid preparations may contain colouring agents, flavouring agents, saccharine and/or carboxymethylcellulose as a thickening agent or other excipients known to those skilled in art.

The compounds of the invention may also be administered in conjunction with other compounds used for the treatment of the above conditions.

The term 'medical therapy' as used herein is intended to include prophylactic, diagnostic and therapeutic regimens carried out in vivo or ex vivo on humans or other mammals.

The pharmaceutical compositions may be administered topically (e.g. to the lung and/or airways or to the skin) in the form of solutions, suspensions, heptafluoroalkane aerosols and dry powder formulations, or systemically, e.g. by oral administration in the form of tablets, capsules, syrups, powders or granules, or by parenteral administration in the form of solutions or suspensions, or by subcutaneous administration or by rectal administration in the form of suppositories or transdermally. Preferably the compound of the invention is administered orally.

The invention further relates to combination therapies wherein a compound of the invention or a pharmaceutically acceptable salts or solvate thereof, or a pharmaceutical composition or formulation comprising a compound of formula (1) is administered concurrently or sequentially with therapy and/or an agent for the treatment of any one of asthma, allergic rhinitis, cancer, COPD, rheumatoid arthritis, psoriasis, inflammatory bowel diseases, osteoarthritis or osteoporosis.

In particular, for the treatment of the inflammatory diseases rheumatoid arthritis, psoriasis, inflammatory bowel disease, COPD, asthma and allergic rhinitis the compounds of the invention may be combined with agents such as TNF-α inhibitors such as anti-TNF monoclonal antibodies (such as Remicade, CDP-870 and D₂E₇ and TNF receptor immunoglobulin molecules (such as Enbrel®), non-selective COX-1 / COX-2 inhibitors (such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin), COX-2 inhibitors (such as meloxicam, celecoxib, rofecoxib, valdecoxib and etoricoxib) low dose methotrexate, lefunomide, ciclesonide, hydroxychloroquine, d-penicillamine, auranofin or parenteral or oral gold.

The present invention still further relates to the combination of a compound of the invention together with a leukotriene biosynthesis inhibitor, 5-lipoxygenase (5-LO) inhibitor or 5-lipoxygenase activating protein (FLAP) antagonist such as zileuton, ABT-761, fenleuton, tepoxalin, Abbott-79175, Abbott-85761, N-(5-substituted)-thiophene-2-alkylsulfonamides, 2,6-di-tert-butylphenol hydrazones, methoxytetrahydropyrans such as Zeneca ZD-2138, the compound SB-210661, pyridinyl-substituted 2-cyanonaphthalene compounds such as L-739,010,2-cyanoquinoline compounds such as L-746,530, indole and quinoline compounds such as MK-591, MK-886, and BAY x 1005.

The present invention still further relates to the combination of a compound of the invention together with a receptor antagonist for leukotrienes LTB₄, LTC₄, LTD₄, and LTE₄ selected from the group consisting of the phenothiazin-3-ones such as L-651,392, amidino compounds such as CGS-25019c, benzoxalamines such as ontazolast, benzenecarboximidamides such as BIBL 284/260, and compounds such as zafirlukast, ablukast, montelukast, pranlukast, verlukast (MK-679), RG-12525, Ro-245913, iralukast (CGP 45715A), and BAY x 7195.

The present invention still further relates to the combination of a compound of the invention together with a PDE4 inhibitor including inhibitors of the isoform PDE4D.

The present invention still further relates to the combination of a compound of the invention together with a antihistaminic H₂ receptor antagonists such as cetirizine, loratadine, desloratadine, fexofenadine, astemizole, azelastine, and chlorpheniramine.

The present invention still further relates to the combination of a compound of the invention together with a gastroprotective H₂. receptor antagonist.

The present invention still further relates to the combination of a compound of the invention together with an α₁.- and α₂.-adrenoceptor agonist vasoconstrictor sympathomimetic agent, such as propylhexedrine, phenylephrine, phenylpropanolamine, pseudoephedrine, naphazoline hydrochloride, oxymetazoline hydrochloride, tetrahydrozoline hydrochloride, xylometazoline hydrochloride, and ethylnorepinephrine hydrochloride.

The present invention still further relates to the combination of a compound of the invention together with anticholinergic agents such as ipratropium bromide, tiotropium bromide, oxitropium bromide, pirenzepine, and telenzepine.

The present invention still further relates to the combination of a compound of the invention together with a β₁- to β₄-adrenoceptor agonists such as metaproterenol, isoproterenol, isoprenaline, albuterol, salbutamol, formoterol, salmeterol, terbutaline, orciprenaline, bitolterol mesylate, and pirbuterol, or methylxanthanines including theophylline and aminophylline, sodium cromoglycate, or muscarinic receptor (M1, M2, and M3) antagonist.

The present invention still further relates to the combination of a compound of the invention together with an insulin-like growth factor type I (IGF-1) mimetic.

The present invention still further relates to the combination of a compound of the invention together with an inhaled glucocorticoid with reduced systemic side effects, such as prednisone, prednisolone, flunisolide, triamcinolone acetonide, beclomethasone dipropionate, budesonide, fluticasone propionate, and mometasone furoate.

The present invention still further relates to the combination of a compound of the invention together with an inhibitor of matrix metalloproteases (MMPs), i.e., the stromelysins, the collagenases, and the gelatinases, as well as aggrecanase, especially collagenase-1 (MMP-1), collagenase-2 (MMP-8), collagenase-3 (MMP-13), stromelysin-1 (MMP-3), stromelysin-2 (MMP-10), and stromelysin-3 (MMP-11) and MMP-12.

The present invention still further relates to the combination of a compound of the invention together with other modulators of chemokine receptor function such as CCR1, CCR2, CCR2A, CCR2B, CCR3, CCR4, CCR5, CCR6, CCR7, CCR8, CCR9, CCR10 and CCR11 (for the C-C family), CXCR1, CXCR3, CXCR4 and CXCR5 (for the C-X-C family) and CX₃CR1 for the C-X₃-C family.

The present invention still further relates to the combination of a compound of the invention together with antiviral agents such as Viracept, AZT, aciclovir and famciclovir, and antisepsis compounds such as Valant.

The present invention still further relates to the combination of a compound of the invention together with cardiovascular agents such as calcium channel blockers, lipid lowering agents such as statins, fibrates, beta-blockers, Ace inhibitors, Angiotensin-2 receptor antagonists and platelet aggregation inhibitors.

The present invention still further relates to the combination of a compound of the invention together with CNS agents such as antidepressants (such as sertraline), anti-Parkinsonian drugs (such as deprenyl, L-dopa, Requip, Mirapex, MAOB inhibitors such as selegine and rasagiline, comP inhibitors such as Tasmar, A-2 inhibitors, dopamine reuptake inhibitors, NMDA antagonists, Nicotine agonists, Dopamine agonists and inhibitors of neuronal nitric oxide synthase), and anti-Alzheimer's drugs such as donepezil, tacrine, COX-2 inhibitors, propentofylline or metryfonate.

The present invention still further relates to the combination of a compound of the invention together with (i) tryptase inhibitors, (ii) platelet activating factor (PAF) antagonists, (iii) interleukin converting enzyme (ICE) inhibitors, (iv) IMPDH inhibitors, (v) adhesion molecule inhibitors including VLA-4 antagonists, (vi) cathepsins, (vii) MAP kinase inhibitors, (viii) glucose-6 phosphate dehydrogenase inhibitors, (ix) kinin-B₁- and B₂-receptor antagonists, (x) anti-gout agents, e.g., colchicine, (xi) xanthine oxidase inhibitors, e.g., allopurinol, (xii) uricosuric agents, e.g., probenecid, sulfinpyrazone, and benzbromarone, (xiii) growth hormone secretagogues, (xiv) transforming growth factor (TGFβ), (xv) platelet-derived growth factor (PDGF), (xvi) fibroblast growth factor, e.g., basic fibroblast growth factor (bFGF), (xvii) granulocyte macrophage colony stimulating factor (GM-CSF), (xviii) capsaicin cream, (xix) Tachykinin NK₁ and NK₃ receptor antagonists selected from the group consisting of NKP-608C, SB-233412 (talnetant), and D-4418, (xx) elastase inhibitors selected from the group consisting of UT-77 and ZD-0892, (xxi) TNFα converting enzyme inhibitors (TACE), (xxii) induced nitric oxide synthase inhibitors (iNOS) or (xxiii) chemoattractant receptor-homologous molecule expressed on TH2 cells, (CRTH2 antagonists).

The compounds of the present invention may also be used in combination with osteoporosis agents such as roloxifene, droloxifene, lasofoxifene or fosomax and immunosuppressant agents such as FK-506, rapamycin, cyclosporine, azathioprine, and methotrexate.

The compounds of the invention may also be used in combination with existing therapeutic agents for the treatment of osteoarthritis. Suitable agents to be used in combination include standard non-steroidal anti-inflammatory agents (hereinafter NSAID's) such as piroxicam, diclofenac, propionic acids such as naproxen, flubiprofen, fenoprofen, ketoprofen and ibuprofen, fenamates such as mefenamic acid, indomethacin, sulindac, apazone, pyrazolones such as phenylbutazone, salicylates such as aspirin, COX-2 inhibitors such as celecoxib, valdecoxib, rofecoxib and etoricoxib, analgesics and intraarticular therapies such as corticosteroids and hyaluronic acids such as hyalgan and synvisc and P2X7 receptor antagonists.

The compounds of the invention can also be used in combination with existing therapeutic agents for the treatment of cancer. Suitable agents to be used in combination include:
(i) antiproliferative/antineoplastic drugs and combinations thereof, as used in medical oncology, such as alkylating agents (for example cis-platin, carboplatin, cyclophosphamide, nitrogen mustard, melphalan, chlorambucil, busulphan and nitrosoureas), antimetabolites (for example antifolates such as fluoropyrimidines like 5-fluorouracil and tegafur, raltitrexed, methotrexate, cytosine arabinoside, hydroxyurea, gemcitabine and paclitaxel (Taxol®), antitumour antibiotics (for example anthracyclines like adriamycin, bleomycin, doxorubicin, daunomycin, epirubicin, idarubicin, mitomycin-C, dactinomycin and mithramycin), antimitotic agents (for example vinca alkaloids like vincristine, vinblastine, vindesine and vinorelbine and taxoids like taxol and taxotere), and topoisomerase inhibitors (for example epipodophyllotoxins like etoposide and teniposide, amsacrine, topotecan and camptothecin),
(ii) cytostatic agents such as antioestrogens (for example tamoxifen, toremifene, raloxifene, droloxifene and iodoxyfene), oestrogen receptor down regulators (for example fulvestrant), antiandrogens (for example bicalutamide, flutamide, nilutamide and cyproterone acetate), LHRH antagonists or LHRH agonists (for example goserelin, leuprorelin and buserelin), progestogens (for example megestrol acetate), aromatase inhibitors (for example as anastrozole, letrozole, vorazole and exemestane) and inhibitors of 5α-reductase such as finasteride,
(iii) Agents which inhibit cancer cell invasion (for example metalloproteinase inhibitors like marimastat and inhibitors of urokinase plasminogen activator receptor function),
(iv) inhibitors of growth factor function, for example such inhibitors include growth factor antibodies, growth factor receptor antibodies (for example the anti-erbb2 antibody trastuzumab [Herceptin^{™}] and the anti-erbb1 antibody cetuximab [C225]), farnesyl transferase inhibitors, tyrosine kinase inhibitors and serine/threonine kinase inhibitors, for example inhibitors of the epidermal growth factor family (for example EGFR family tyrosine kinase inhibitors such as N-(3-chloro-4-fluorophenyl)-7-methoxy-6-(3-morpholinopropoxy)quinazolin-4-amine (gefitinib, AZD1839), N-(3-ethynylphenyl)-6,7-bis(2-methoxyethoxy)quinazolin-4-amine (erlotinib, OSI-774) and 6-acrylamido-N-(3-chloro-4-fluorophenyl)-7-(3-morpholinopropoxy)quinazolin-4-amine (CI 1033)), for example inhibitors of the platelet-derived growth factor family and for example inhibitors of the hepatocyte growth factor family,
(v) antiangiogenic agents such as those which inhibit the effects of vascular endothelial growth factor, (for example the anti-vascular endothelial cell growth factor antibody bevacizumab [Avastin^{™}], compounds such as those disclosed in International Patent Applications WO 97/22596, WO 97/30035, WO 97/32856 and WO 98/13354) and compounds that work by other mechanisms (for example linomide, inhibitors of integrin αvβ3 function and angiostatin),
(vi) vascular damaging agents such as Combretastatin A4 and compounds disclosed in International Patent Applications WO 99/02166, WO00/40529, WO 00/41669, WO01/92224, WO02/04434 and WO02/08213,
(vii) antisense therapies, for example those which are directed to the targets listed above, such as ISIS 2503, an anti-ras antisense,
(viii) gene therapy approaches, including for example approaches to replace aberrant genes such as aberrant p53 or aberrant BRCA 1 or BRCA2, GDEPT (gene-directed enzyme pro-drug therapy) approaches such as those using cytosine deaminase, thymidine kinase or a bacterial nitroreductase enzyme and approaches to increase patient tolerance to chemotherapy or radiotherapy such as multi-drug resistance gene therapy, and
(ix) immunotherapy approaches, including for example ex-vivo and in-vivo approaches to increase the immunogenicity of patient tumour cells, such as transfection with cytokines such as interleukin 2, interleukin 4 or granulocyte-macrophage colony stimulating factor, approaches to decrease T-cell anergy, approaches using transfected immune cells such as cytokine-transfected dendritic cells, approaches using cytokine-transfected tumour cell lines and approaches using anti-idiotypic antibodies.

The following Examples illustrate the invention.

*General methods* All reactions were performed in dried glassware in an argon atmosphere at room temperature, unless otherwise noted. All solvents and reagents and solvents were used as received. Merck Silica gel 60 (0.040-0.063 mm) was used for preparative silica gel chromatography. A Kromasil KR-100-5-C18 column (250 x 20 mm, Akzo Nobel) and mixtures of acetonitrile/water at a flow rate of 10 ml/min was used for preparative HPLC. Reactions were monitored at 254 nm by analytical HPLC, using a Kromasil C-18 column (150 x 4.6 mm) and a gradient (containing 0.1 % trifluoroacetic acid) of 5 to 100% of acetonitrile in water at a flow rate of I ml/min. Evaporations of solvents were performed under reduced pressure using a rotary evaporator at a maximum temperature of 40°C. Products were dried under reduced pressure at 40 °C.
¹H-NMR spectra were recorded on a Varian Inova-400 or Unity-500+ instrument. The central solvent peak of chloroform-*d* (δ_{H} 7.27 ppm), dimethylsulfoxide-*d₆* (δ_{H} 2.50 ppm) or methanol-*d*₄ (δ_{H} 3.35 ppm) were used as internal references. Low resolution mass spectra obtained on a Hewlett Packard 1100 LC-MS system equipped with a APCI ionisation chamber.

Merck Silica gel 60 (0.040-0.063 mm) was used for preparative silica gel chromatography. A Kromasil KR-100-5-C18 column (250 x 20 mm, Akzo Nobel) and mixtures of acetonitrile/water at a flow rate of 10 ml/min was used for preparative HPLC. Reactions were monitored at 254 nm by analytical HPLC, using a Kromasil C-18 column (150 x 4.6 mm) and a gradient (containing 0.1 % trifluoroacetic acid) of 5 to 100% of acetonitrile in water at a flow rate of 1 ml/min. Evaporations of solvents were performed under reduced pressure using a rotary evaporator at a maximum temperature of 40°C. Products were dried under reduced pressure at 40°C.

### Example 1

### 4-(2-Ethyl-phenylamino)-2-(4-fluorophenyl)-1H-pyrrolo[2,3-b]pyridine-5-carboxylic acid amide

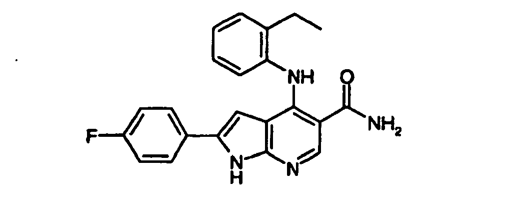

### a) 6-Amino-5-iodo-4-methoxy-nicotinic acid methyl ester

In a 250 ml roundbottomed flask was dissolved 6-amino-4-methoxy nicotinic acid methyl ester (1.5 g, 8.28 mmol, prepared according to literature procedures) in 165 ml methanol. To this stirred solution was added Iodine (6.3 g, 24.8 mmol) and Silver trifluoroacetate (4.91 g, 22.3 mmol). The mixture was stirred in darkness at room temperature for 48 hours, and an almost complete conversion of the starting material was observed. The mixture was diluted to the double volume by the addition of methanol, and was then filtered through Celite ®**,** and the filter cake was washed with methanol. All the filtrates were combined, and concentrated in vaccuo, giving a dark red-brown residue. This residue was taken up in CH₂Cl₂ (300 ml) and was washed with a water solution of sodium thiosulfate (10% in water), and the organic phase was decolorized. The organic phase was thereafter washed with Brine, and dried over Na₂SO₄. The organic solvent was finally removed in vaccuo. Purification on silica (Heptane : EtOAc 3:1 to 2:1) provided 1.5 g (59 %) of the sub-title compound.

¹H-NMR (400 MHz, DMSO-*d6*): δ 8.33 (s, 1H), 6.89 (bs, 2H), 3.76 (s, 3H), 3.75 (s, 3H)

### b) 6-Amino-5-(4-fluoro-phenylethynyl)-4-methoxy-nicotinic acid methyl ester

In a 250 ml roundbottomed flask was dissolved the compound obtained in a (1.9 g, 6.16 mmol) in THF (14 ml) and triethylamine (85 ml). The solution was degassed by bubbling a stream of nitrogen through the solution for 5 minutes. To this solution was subsequently added Pd(PPh₃)₂Cl₂ (0.14 g, 0.2 mmol), CuI (0.05 g, 0.26 mmol) and 4-Ethynylfluorobenzene (0.85 g, 7.07 mmol). The flask was sealed and heated with stirring for 30 minutes at 60°C. Analysis by LC-MS showed 80% conversion. Additional amounts of 4-Ethynyl-fluorobenzene (0.05 g, 0.4 mmol) and Pd(PPh₃)₂Cl₂ (0.02 g, 0.03 mmol) was added, and the reaction was stirred for another 30 minutes, and complete conversion was observed. The mixture was allowed to cool, and was then concentrated in vaccuo giving a crude product. The material was purified on silica (Heptane : EtOAc 2:1), giving 1.7 g (92%) of the sub-title compound as a yellowish solid.

H-NMR (400 MHz, DMSO-*d6*): δ 8.37 (s, 1H), 7.72 (dd, 2H, *J* 8.96 Hz), 7.27 (t, 2H, *J* 8.96 Hz), 7.13 (bs, 2H), 3.97 (s, 3H), 3.75 (s, 3H)

### c) 2-(4-Fluoro-phenyl)-4-methoxy-1H-pyrrolo[2,3-b]pyridine-5-carboxylic acid methyl ester

In a 25 ml roundbottomed flask was dissolved the compound obtained in b (0.46 g, 1.53 mmol) in NMP (15 ml, dried over mol. sieves). To the stirred solution was added KOBu^{t} (0.56 g, 4.68 mmol), and the flask was sealed, and heated (40°C) with stirring for 4 hours, following the reaction on TLC ( DCM : MeOH 99:1 on silica plates). When complete reaction was observed, the reaction was allowed to cool, and was then partitioned between EtOAc (100 ml) and 0.5M aqueous hydrochloric acid (100 ml). The organic phase was collected, and the aqueous phase was extracted with another portion of EtOAc (50 ml) The combined organic phases were washed with water (5 x 40 ml) and Brine (20 ml) and then dried over Na₂SO₄. Filtration and subsequent evaporation gave a solid. To this solid was added ether (50 ml) and the inhomogeneous mixture was stirred for 10 minutes, and the solid product was then isolated by filtration giving 0.39 g (86%) of a slightly yellowish solid.

H-NMR (400 MHz, DMSO-*d*6): δ 12.49 (s, 1H), 8.47 (s, 1H), 8.02 (dd, 2H *J* 8.90 Hz), 7.40 (d, 1H, *J* 2.04 Hz), 7.30 (t, 2H, *J* 8.90 Hz), 4.34 (s, 3H), 3.80 (s, 3H)

### d) 2-(4-Fluoro-phenyl)-4-hydroxy-1H-pyrrolo[2,3-b]pyridine-5-carboxylic acid

In a pressure safe glass vessel was added the compound obtained in c (0.91 g, 3.03 mmol) and aqueous hydrobromic acid (10 ml, 48% in water) and a magnetic stirrer. The vessel was sealed and the mixture was heated (120°) with stirring for 4 hours. LC-MS confirmed the complete conversion of the starting material, and the mixture was allowed to cool. The mixture was diluted to the double water by addition of water. The insoluble product was isolated by filtration, and the solid was washed with water on the filter, and was then dried with air, giving 0.74 g (90%) of the sub-title compound as a white powder.

H-NMR (400 MHz, DMSO-*d6*: δ 12.57 (s, 1H), 8.38 (s, 1H), 7.90 (dd, 2H *J* 8.77 Hz), 7.31 (t, 2H *J* 8.80 Hz), 7.06 (d, 1H *J* 2.18 Hz)

### e) 4-Chloro-2-(4-fluoro-phenyl)-1H-pyrrolo[2,3-b]pyridine-5-carboxylic acid amide

In a pressure safe glass vessel was added the compound obtained in d (0.74 g, 2.72 mmol) and POCl₃ and a magnetic stirrer. The vessel was sealed and heated (100°C) with stirring for 2 hours. The reaction was monitored on LC-MS, by taking out a drop from the solution which was evaporated and then quenched with methanol. The product was analyzed as the methyl ester. When complete conversion was observed, the volatile components were removed in vaccuo, giving a yellow solid. The solid was dissolved in dry 1,4-Dioxane (10 ml), and the stirred solution was cooled on an ice bath. Ammonia (32% aqueous solution, 2 ml) was added immediately giving a exothermic reaction. The resulting mixture was stirred for 5 minutes, and the product precipitated. The crude mixture was evaporated to dryness, giving a solid. To the solid was added water (10 ml), and the mixture was stirred for 10 minutes. The insoluble product was isolated by filtration and was washed on the filter with water, and was finally air-dried, giving 0.67 g (85%) of the sub-title compound as an off-white solid.

H-NMR (400 MHz, DMSO-*d6*): δ 12.64 (s, 1H), 8.29 (s, 1H), 8.07 (dd, 2H *J* 8.80 Hz). 7.92 (bs, 1H), 7.63 (bs, 1H), 7.35 (t, 2H *J* 8.86 Hz), 7.06 (d, 1H *J* 2.1 Hz)

### 4-(2-Ethyl-phenylamino)-2-(4-fluorophenyl)-1H-pyrrolo[2,3-b]pyridine-5-carboxylic acid amide

In a microwave sample vessel was added the compound obtained in e (0.05 g, 0.173 mmol), 2-Ethylaniline (0.10 g, 0.83 mmol) and NMP (2 ml) and a magnetic stirrer. The vessel was sealed and was heated in the microwave reactor (170°C, 40 minutes). Analysis of the resulting mixture showed complete conversion of compound e. The solution was diluted with water and 1,4-Dioxane and was then purified on preparative HPLC. Lyophilization of pure fractions gave 0.03 g of the Trifluoroacetic acid salt of tho title compound. Extraction between EtOAc and alkaline water solution gave the neutral form of the title compound. 0.025 g (39%) was obtained of a white solid.

H-NMR (400 MHz, DMSO-*d6*): δ 12.05 (s, 1H), 11.09 (s, 1H), 8.55 (s, 1H), 8.05 (bs, 1H), 7.49 (dd, 2H *J* 8.44 Hz), 7.38 (d, 1H *J* 7.33 Hz), 7.33-7.17 (m, 6H), 5.39 (d. 1H *J* 2.00 Hz), 2.61 (q, 2H *J* 7.41 Hz), 1.13 (t, 3H *J* 7.50 Hz)

### Example 2

### 4-(2-Ethyl-3-hydroxymethyl-phenylamino)-2-(4-fluorophenyl)-1H-pyrrolo[2,3-b]pyridine-5-carboxylic acid amide trifluoro acetic acid salt

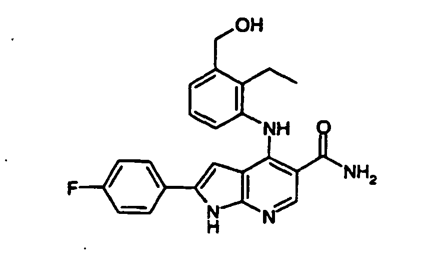

In a microwave reaction vessel was added the compound obtained in Example le (0.10 g, 0.348 mmol) and (3-Amino-2-ethyl-phenyl)-methanol (0.104 g, 0.692 mmol). To this mixture of solids were added Ethoxyethanol (2 ml) and Pyridine hydrochloride (0.04 g, 0.346 mmol). The vessel was sealed, and heated in the microwave reactor (170°C, 45 minutes), when almost complete conversion of the chlorine containing starting material was observed. The volatile solvent was removed in vaccuo, and the residue was dissolved in a mixture of 1,4-Dioxane (2.5 ml) and water (1.5 ml) and 5 drops of TFA. The mixture was purified on preparative HPLC giving 0.04 g (22%) of a white solid after lyophilization of the pure fractions.

H-NMR (400 MHz, DMSO-*d6*): δ 12.03 (s, 1H), 11.14 (s, 1H), 8.53 (s, 1H), 8.03 (bs, 1H), 7.49 (dd, 2H *J* 8.97 Hz), 7.36 (d, 1H *J 7.43* Hz), 7.30 (bs. 1H), 7.24-7.16 (m, 3H), 7.09 (d, 1H *J* 7.69 Hz), 5.45 (d, 1H *J* 2.05 Hz), 5.20 (t, 1H *J* 5.32 Hz), 4.61 (d, 2H *J* 4.94 Hz), 2.66 (q, 2H *J* 7.82 Hz), 1.07 (t, 3H *J* 7.66 Hz)

APCI-MS m/z 405.3 [MH+]

### Example 3

### 4-{2-Ethyl-3-[(2-hydroxy-ethylamino)-methyl]-phenylamino}-2-(4-fluoro-phenyl)-1H-pyrrolo[2,3-b]pyridine-5-carboxylic acid amide trifluoroacetic acid salt

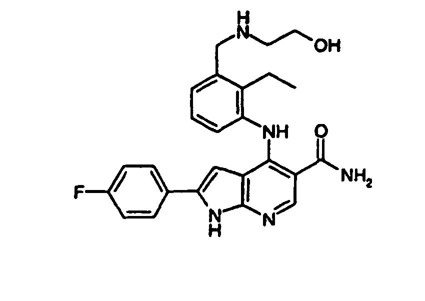

In a 10 ml roundbottomed flask was dissolved the compound obtained in Example 2 (0.01g, 19.3 µmol) in CH₂Cl₂ (5 ml, dried over mol.sieves). To this solution was added SOCl₂ (0.03g, 0.25 mmol) and a magnetic stirrer. The flask was sealed and stirred for 1 hour in room temperature, and LC-MS showed a complete conversion to the benzyl chloride. The volatiles were removed in vaccuo, and the residue was dissolved in NMP (1.5 ml), and transferred to a microwave reaction vessel. To this solution was added 2-Aminoethanol (0.03, 0.5 mmol) and a magnetic stirrer, and the mixture were heated in the microwave reactor (90°C, 15 minutes). LC-MS on the resulting mixture confirmed the complete conversion to the desired product. The mixture was diluted to the double volume with water and acidified with TFA, and was then purified on preparative HPLC. Lyophilization of pure fractions gave 0.01 g (92%) of the title compound.

H-NMR (400 MHz, DMSO-*d*6): δ 12.03 (s, 1H), 11.14 (s, 1H), 8.54 (s, 1H), 8.03 (bs, 1H), 7.49 (dd, 2H *J* 8.83 Hz), 7.34 (d, 1H *J* 7.68 Hz), 7.30 (bs, 1H), 7.23-7.16 (m, 3H), 7.09 (d, 1H *J* 7.68 Hz), 5.39 (d, 1H *J* 1.51 Hz), 4.52 (t, 1H *J* 5.47 Hz), 3.80 (s, 2H), 3.49 (q, 2H *J* 5.53 Hz), 2.72 (q, 2H *J* 7.62 Hz), 2.65 (t, 2H *J* 5.75 Hz), 1.09 (t, 3H *J* 7.65 Hz)

APCI-MS m/z 448.3 [MH+]

### Example 4

### 4-(2-Ethyl-3-{[(2-hyroxy-ethyl)-methyl-amino]-methyl}-phenylamino)-2-(4-fluorophenyl)-1H-pyrrolo[2,3-b]pyridine-5-carboxylic acid amide Trifluoroacetic acid salt

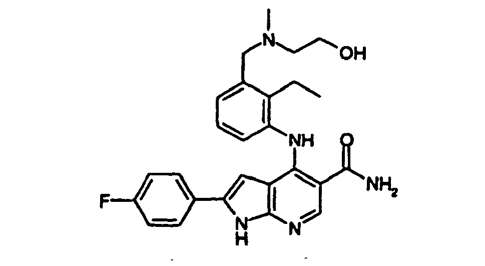

The compound was prepared according to the procedure in Example 3, obtaining 0.005 g (75%) of the title compound.

APCI-MS m/z 462.3 [MH+] for the free amine.

### Example 5

### 4-{2-Ethy1-3-[(2-hydroxy-1-methyl-ethylamino)-methyl]-phenylamino}-2-(4-fluorophenyl)-1H-pyrrolo[2,3-b]pyridine-5-carboxylic acid amide Trifluoroacetic acid salt

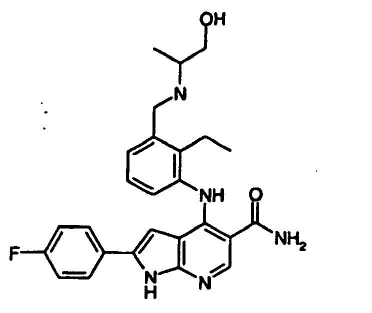

The compound was prepared according to the procedure in Example 3, obtaining 0.004 g (60%) of the title compound.

APCI-MS m/z 462.3 [MH+] for the free amine.

### Example 6

### 4-{2-Ethyl-3-((S)-(2-hydroxy-1-phenyl-ethylamino)-methyl]-phenylamino}-2-(4-fluoro-phenyl)-1H-pyrrolo[2,3-b]pyridine-5-carboxylic acid amide -Trifluoroacetic acid salt

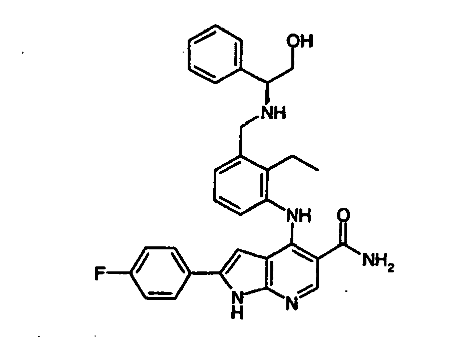

The compound was prepared according to the procedure in Example 3, obtaining 0.004 g (65%) of the title compound.

APCI-MS m/z 524.3 [MH+] for the free amine.

### Example 7

### 4-{2-Ethyl-3-[(2-hydroxy-2-phenyl-ethylamino)-methyl]-phenylamino}-2-(4-fluorophenyl)-1H-pyrrolo[2,3-b]pyridine-5-carboxylic acid amide Trifluoroacetic acid salt

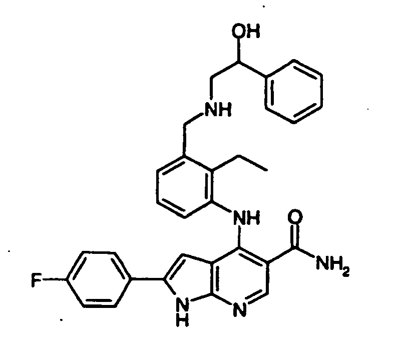

The compound was prepared according to the procedure in Example 3, obtaining 0.005 g (67%) of the title compound.

APCI-MS m/z 524.3 [MH+] for the free amine.

### Example 8

### 4-(2-Ethyl-3-morpholin-4-ylmethyl-phenylamino)-2-(4-fluoro-phenyl)-1H-pyrrolo[2,3-b]pyridine-5-carboxylic acid amide Trifluoroacetic acid salt

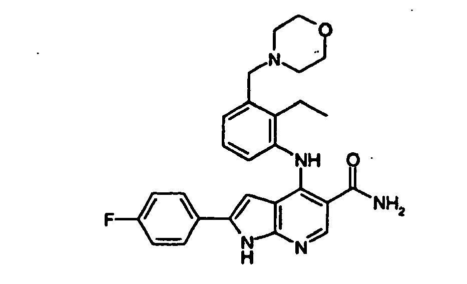

The compound was prepared according to the procedure in Example 3, obtaining 0.003 g (53%) of the title compound.

APCI-MS m/z 474.2 [MH+] for the free amine.

### Example 9

### 4-[2-Ethyl-3-(3-hydroxy-pyrrolidin-1-ylmethyl)-phenylamino]-2-(4-fluoro-phenyl)-1H-pyrrolo[2,3-b]pyridine-5-carboxylic acid amide Trifluoroacetic acid salt

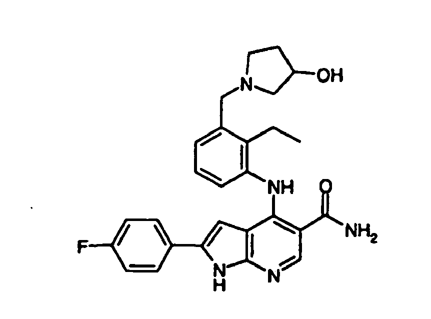

The compound was prepared according to the procedure in Example 3, obtaining 0.004 g (71 %) of the title compound.

APCI-MS m/z 474.2 [MH+] for the free amine.

### Example 10

### 4-[2-Ethyl-3-((R)-2-hydroxymethyl-pyrrolidin-1-ylmethyl)-phenylamino]-2-(4-fluorophenyl)-1H-pyrrolo[2,3-b]pyridine-5-carboxylic acid amide Trifluoroacetic acid salt

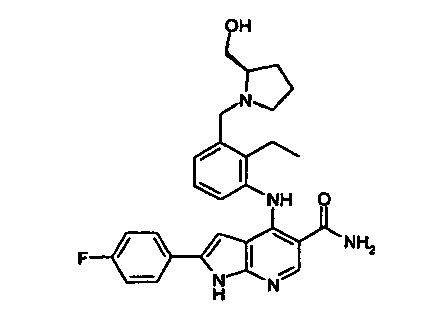

The compound was prepared according to the procedure in Example 3, obtaining 0.003 g (52%) of the title compound.

APCI-MS m/z 488.4 [MH+] for the free amine.

### Example 11

### 4-{3-[(2,3-Dihydroxy-propylamino)-methyl]-2-ethyl-phenylamino}-2-(4-fluorophenyl)-1H-pyrrolo[2,3-b]pyridine-5-carboxylic acid amide Trifluoroacetic acid salt

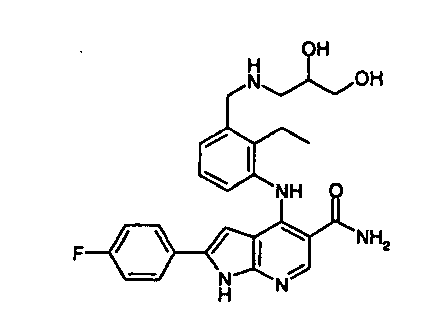

The compound was prepared according to the procedure in Example 3, obtaining 0.005 g (87%) of the title compound.

APCI-MS m/z 478.3 [MH+] for the free amine.

### Example 12

### 4-(2-Ethyl-3-imidazol-1-ylmethyl-phenylamino)-2-(4-fluoro-phenyl)-1H-pyrrolo[2,3-b]pyridine-5-carboxylic acid amide Trifluoroacetic acid salt

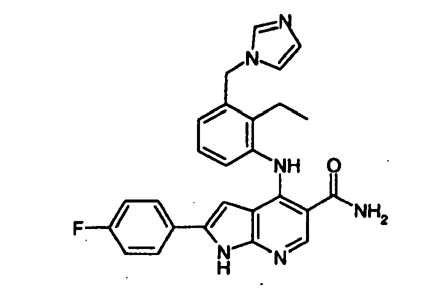

The compound was prepared according to the procedure in Example 3, with the exception that the temperature was 110°C, and the reaction time was 30 minutes. The outcome of the synthesis was 0.004 g (73%) of the title compound.

APCI-MS m/z 455.3 [MH+] for the free amine.

### Example 13

### 4-[3-(2-Ethoxy-ethoxymethyl)-2-ethyl-phenylamino]-2-(4-fluoro-phenyl)-1H-pyrrolo[2,3-b]pyridine-5-carboxylic acid amide

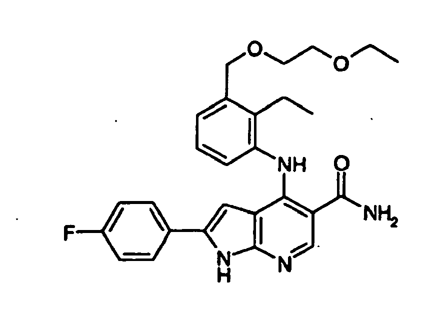

The substans was obtained as a by-product in the reaction described in Example 2. The product was isolated by preparative HPLC. Pure fractions were lyophilized, giving the TFA salt as a yellowish solid. The free amine was obtained by extraction between EtOAc and 1M NaOH. The organic phase was dried, and evaporated, giving 0.035 g (21%) of a yellow solid.

H-NMR (400 MHz, DMSO-*d6*): δ 12.05 (s, 1H), 11.17 (s, 1H), 8.55 (s, 1H), 8.05 (bs, 1H), 7.50 (dd, 2H *J* 8.62 Hz), 7.33 (d, 1H *J* 7.58 Hz), 7.32 (bs, 1H), 7.26-7.14 (m, 4H), 5.42 (d, 1H *J* 2.10 Hz), 4.60 (s, 2H), 3.63-3.59 (m, 2H), 3.55-3.51 (m, 2H), 3.42 (q, 2H *J* 6.82 Hz), 2.74-2.64 (m, 2H), 1.13-1.06 (m, 6H)

### Example 14

### 2-(4-Bromo-phenyl)-4-(2-ethyl-phenylamino)-1H-pyrrolo[2,3-b]pyridine-5-carboxylic acid amide

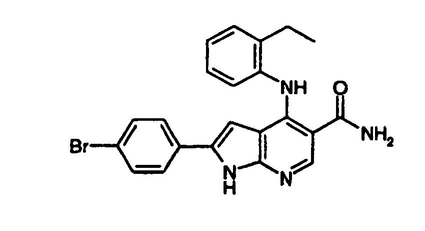

The compound was prepared according to the procedure described in Example 1, with the exception that this product was purified on silica (CH₂Cl₂ : MeOH 99:1 to 98 : 2 to 97 to 3). 0.04 g was prepared.

H-NMR (400 MHz, DMSO-*d6)*: δ 12.10 (s, 1H), 11.13 (s, 1H), 8.56 (s, 1H), 8.05 (bs, 1H), 7.55 (d, 2H *J* 8.88 Hz), 7.43-7.37 (m, 3H), 7.33-7.23 (m, 2H), 7.30 (bs, 1H), 7.21 (d, 1H *J* 7.54 Hz), 5.46 (d, 1H *J* 2.0 Hz), 2.61 (q, 2H *J* 7.46 Hz), 1.13 (t, 3H *J* 7.45 Hz)

### Example 15

### 4-(2-Ethyl-phenylamino)-2-phenyl-1H-pyrrolo[2,3-b]pyridine-5-carboxylic acid amide

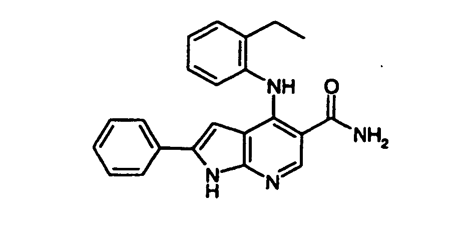

The compound was prepared according to the procedure described in Example 1, and was purified according to the procedure in Example 14, giving 0.007g of the title compound as a white solid.

H-NMR (400 MHz, DMSO-*d6*): δ 12.04 (s, 1H), 11.09 (s, 1H), 8.55 (s, 1H), 8.03 (bs, 1H), 7.47 (d, 2H *J* 8.19 Hz), 7.41-7.20 (m, 8H), 5.44 (d, 1H *J* 2.10 Hz), 2.61 (q, 2H *J* 7.62 Hz), 1.14 (t, 3H *J* 7.70)

APCI-MS m/z 357.3 [MH+]

### Example 16

### 4-(2-Ethyl-3-hydroxymethyl-phenylamino)-2-phenyl-1H-pyrrolo[2,3-b]pyridine-5-carboxylic acid amide Trifluoroacetic acid salt

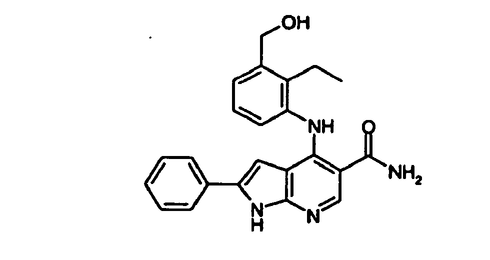

The compound was prepared according to the procedure described in Example 2.

APCI-MS m/z 387.2 [MH+]

### Example 17

### 2-(4-Chloro-phenyl)-4-(2-ethyl-3-hydroxymethyl-phenylamino)-1H-pyrrolo[2,3-b]pyridine-5-carboxylic acid amide Trifluoroacetic acid salt

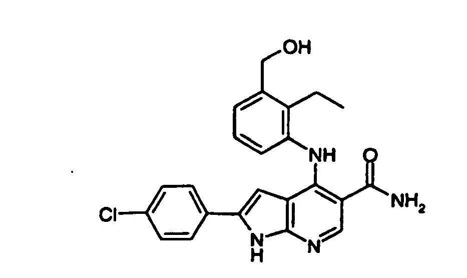

The title compound was prepared according to the procedure described in Example 2. NMR was run on the TFA salt, which give other shifts for acidic protons.

H-NMR (400 MHz, DMSO-*d*₆): δ 12.40 (bs, 1H), 11.44 (bs, 1H), 8.57 (s, 1H), 8.19 (bs, 1H), 7.57-7.40 (m, 7H), 7.27 (t, 1H *J* 7.68 Hz), 7.15 (d, 1H *J* 7.70 Hz), 5.46 (d, 1H *J* 1.90 Hz), 4.63 (s, 2H), 2.71-2.60 (m, 2H), 1.07 (t, 3H *J* 7.63 Hz)

APCI-MS m/z 421.2 [MH+]

### Example 18

### 2-(4-Chloro-phenyl)-4-(2-ethyl-3-imidazol-1-ylmethyl-phenylamino)-1H-pyrrolo[2,3-b]pyridine-5-carboxylic acid amide Trifluoroacetic acid salt

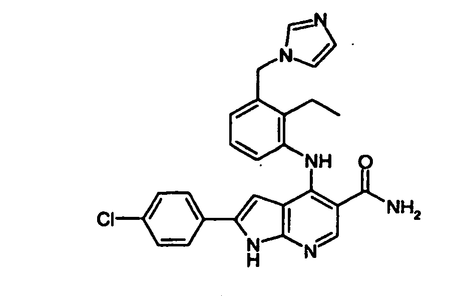

The tiltle compound was prepared according to the procedure in Example 12.

APCI-MS m/z 471.0 [MH+]

### Example 19

### 4-(2-Ethyl-phenylamino)-1H-pyrrolo[2,3-b]pyridine-5-carboxylic acid amide

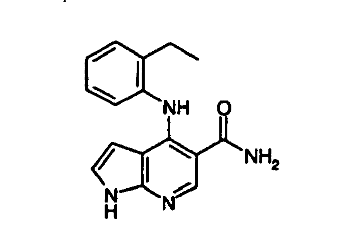

### a) 1-Benzyl-5-nitro-1H-pyrrole-2-carboxylic acid benzyl ester

In a flask was dissolved 5-nitro-1*H*-pyrrole-2-carboxylic acid (0.86 g, 5.51 mmol), in NMP (5 ml). To this solution was added Cs₂CO₃ (3.76 g, 11.5 mmol) and Benzyl bromide (1.88 g, 11.02 mmol) and a magnetic stirrer. The mixture was stirred at room temperature for 1.5 hours, and was monitored by TLC, which confirmed the complete conversion of the starting material. The mixture was partitioned between EtOAc (25 ml) and water (25 ml). The organic phase was collected and the water phase was extracted with another portion of EtOAc (20 ml). The combined organic phases were washed with water (2 x 20 ml), and brine (20 ml). The organic phase was then concentrated in vaccuo, giving a crude product, which was purified on silica, giving 0.52 g (28%) of the sub-title compound as an oil, which crystallizes on standing to a white solid.

H-NMR (400 MHz, DMSO-*d*6): δ 8.50 (d, 1H *J* 2.0 Hz), 7.44 (d, 1H *J* 2.0 Hz), 7.39-7.27 (m, 8H), 7.19-7.14 (m, 2H), 5.62 (s, 2H), 5.25 (s, 2H)

### b) 2-[(1-Benzyl-5-benzyloxycarbonyl-1H-pyrrol-2-ylamino)-methylene]-malonic acid diethyl ester

In a 100 ml round-bottomed flask was dissolved the compound obtained in a (0.50 g, 1.48 mmol) in glacial acetic acid (20 ml). To this solution was added 2-Ethoxymethylene malonic acid diethyl ester (0.32 g, 1.48 mmol) and iron powder (1.5 g, 26.8 mmol). The flask was sealed, and was stirred at room temperature over night. This gives a reddish solution with a white precipitate. The suspension was partitioned between EtOAc (200 ml) and water (150 ml). The organic phase was collected, and the aqueous phase was extrected with another portion of EtOAc (150 ml). The combined organic phases were washed with water (2 x 100 ml) and brine (50 ml). The organic solution was concentrated in vaccuo, giving an oil. The oil was purified on silica (Heptane : EtOAc 7:1 to 5:1), giving 0.38 g (54%) of the sub-title compound as an oil, which crystallize on standing to a yellow solid.

H-NMR (400 MHz, CDCl₃): δ 10.91 (d, 1H *J* 13.2 Hz), 8.12 (d, 1H *J* 12.9 Hz), 7.41-7.23 (m, 8H), 7.14-7.08 (m, 3H), 6.02 (d, 1H *J* 4.35 Hz), 5.65 (s, 2H), 5.27 (s, 2H), 4.25 (q, 2H *J* 7.22 Hz), 4.21 (q, 2H *J* 7.20), 1.33 (t, 3H *J* 7.20 Hz), 1.29 (t, 3H *J* 7.20)

### c) 2-[(1-Benzyl-5-carboxy-1H-pyrrol-2-ylamino)-methylene]-malonic acid diethyl ester

In a flask was dissolved the compound obtained in b (0.35 g, 0.74 mmol) in ethanol (25 ml, 99.5%). To this solution was added Pd catalyst (0.08 g, 10% Pd on charcoal). The material was hydrogenated at normal atmospheric pressure and in room temperature for 1 hour, and LC-MS shows complete cleavage of the benzyl ester. The catalyst was removed by filtration through Celite®, and the filtrate was evaporated, to give the sub-title compound as a yellow solid.

H-NMR (400 MHz, CDCl₃): δ 10.99 (d, 1H *J* 13.2 Hz), 8.15 (d, 1H *J* 13.2 Hz), 7.36-7.25 (m, 3H), 7.20-7.14 (m, 3H), 6.07 (d. 1H *J* 4.21 Hz), 5.66 (s, 2H), 4.27 (q, 2H *J* 7.4 Hz), 4.23 (q, 2H *J* 7.4 Hz), 1.35 (t, 3H *J* 7.1 Hz), 1.31 (t, 3H *J* 7.1 Hz)

### d) 1-Benzyl-4-hydroxy-1H-pyrrolo[2,3-b]pyridine-5-carboxylic acid ethyl ester

In a vial (10 ml) was added the compound obtained in c (0.32 g, 0.83 mmol) and diphenylmethane (3 ml) and a magnetic stirrer. The open vial was heated with stirring to 240°C for 10 minutes, and gas evolution (C0₂, decarboxylation) was observed during the first 1-2 minutes. After the 10 minutes of heating, the mixture was allowed to cool. LC-MS confirms the conversion of the starting material to a compound with the correct mass. The crude solution was diluted with CHCl₃ (5 ml), and was added onto a silica column and was eluted with Heptane : EtOAc 6:1, giving 0.15 (64%) of the sub-title compound as a yellowish solid.

H-NMR (400 MHz, CDCl₃): δ 11.87 (s, 1H), 8.79 (s, 1H), 7.35-7.25 (m, 3H), 7.24-7.19 (m, 2H), 7.05 (d, 1H *J* 3.60 Hz), 6.70 (d, 1H *J* 3.60), 5.49 (s, 2H), 4.47 (q, 2H *J* 7.12 Hz), 1.45 (t, 3H *J* 7.12 Hz)

### e) 1-Benzyl-4-chloro-1H-pyrrolo[2,3-b]pyridine-5-carboxylic acid amide

In a flask was dissolved the compound obtained in d (0.42 g, 1.4 mmol) in THF (7 ml). To this solution was added 2M NaOH (7 ml, 14 mmol) and water (7 ml). The mixture was heated with stirring (60°C), and was monitored by LC-MS. When complete reaction was observed, the mixture was allowed to cool, and THF was removed in vaccuo. The residual water solution was acidified by the addition of 1M H₂SO₄ (8 ml), and the carboxylic acid precipitated. The material was collected by filtration, and washed on the filter, and finally dried with a stream of air through the filter, giving 0.35 g (93%) of the acid.

The acid was added to a round-bottomned flask together with SOCl₂ (15 ml) and DMF (10 drops). The flask was sealed and stirred at room temperature, and was monitored by LC-MS in a similar way as described in Example le. When complete reaction was observed, the volatiles were removed in vaccuo, giving a solid intermediate, which was dissolved in 1,4-dioxane (15 ml, dry over sieves), and quenched by the addition of ammonia (5 ml, 25% in water). The mixture was stirred for 5 minutes in room temperature, and the volatiles was then removed in vaccuo, giving a white solid. The solid was washed with water on a glass filter, and then dried in air, giving 0.29 g (73%) of the sub-title compound as a white solid.

H-NMR (400 MHz, CDCl₃): δ 8.84 (s, 1H), 7.36-7.29 (m, 4H), 7.25-7.21 (m, 2H), 6.68 (d, 1H *J* 3.56 Hz), 6.53 (bs, 1H), 5.95 (bs, 1H), 5.53 (s, 2H)

### f) 1-Benzyl-4-(2-ethyl-phenylamino)-1H-pyrrolo[2,3-b]pyridine-5-carboxylic acid amide

In a vial was dissolved the compound obtained in e (0.095 g, 0.33 mmol) in NMP (2 ml). To this solution was added 2-ethylanilin (0.16 g, 1.3 mmol) and p-toluenesulfonic acid (1 mg), and a magnetic stirrer. The vial was sealed an was heated (160°C) with stirring over night, which gives complete conversion of the starting material. The mixture was then allowed to cool, and was partitioned between EtOAc (25 ml) and water (25 ml). The organic phase was collected and the aqueous phase was extracted with another portion of EtOAc (15 ml). The combined organic phases were washed with water (2 x 20 ml) and brine (15 ml). The organic phase was concentrated in vaccuo, and the residue purified on silica (CH₂Cl₂: MeOH 97:3), giving 0.06 g (50%) of an almost white solid

H-NMR (400 MHz, CDCl₃): δ 10.95 (s, 1H), 8.63 (s, 1H), 7.36-7.16 (m, 9H), 6.64 (d, 1H, *J* 3.75 Hz), 6.04 (bs, 2H), 5.45 (s, 2H), 5.20 (d, 1H *J* 3.67 Hz), 2.67 (q, 2H, *J* 7.85 Hz), 1.19 (t, 3H, *J* 7.85 Hz)

### 4-(2-Ethyl-phenylamino)-1H-pyrrolo[2,3-b]pyridine-5-carboxylic acid amide

In a flask cooled in dry ice/ethanol bath was condensed ammonia (gas) to a volume of approximately 15 ml. To the cold liquid was added sodium (15 mg, 0.6 mmol), and a dark blue liquid was obtained. This was allowed to stand for 10 minutes under an inert atmosphere. To this liquid was added the compound obtained in f (0.01 g, 20 µmol), and the mixture was allowed to stand for 15 minutes, and the reaction was quenched by the addition of NH₄Cl, and the cooling bath was removed, and the solution allowed to react room temperature. The residue was taken up in EtOAc (20 ml) and water (15 ml). The organic phase was collected, and was washed with water (10 ml) and brine (10 ml), and was then concentrated in vaccuo.. The residue was purified on silica (CH₂Cl₂:MeOH 97:3), which elutes the product. The outcome of the synthesis was 0.005 g (89%) of the title compound as a yellowish solid.

H-NMR (400 MHz, CDCl₃): δ 10.80 (s, 1H), 10.18 (bs, 1H), 8.56 (s, 1H), 7.38-7.31 (m, 2H), 7.26-7.21 (m, 2H), 6.73 (d, 1H *J* 3.67 Hz), 6.24 (bs, 2H), 5.18 (d, 1H *J* 3.62 Hz), 2.64 (q, 2H *J* 7.64 Hz), 1.17 (t, 3H *J* 7.60 Hz)

### Pharmacological Data

### JAK3 HTRF assay

The JAK3 kinase assay utilizes a fusion protein (Jak3 kinase domain fused to Glutathione S-transferase, GST) coexpressed in E.Coli with GroEL/S, and purified by affinity chromatography on Glutathione Sepharose. The enzyme is diluted in 10 mM Tris-HCl, 150 mM NaCl, 5% mannitol, 2 mM 2-mercaptoetanol and 30% glycerol. The substrate in the kinase reaction is a biotinylated peptide of the autophosphorylation site of JAK3 (biotin-LPDKDYYVVREPG) used at 2 µM. Assay conditions are as follows: JAK3, compound and substrate are incubated in 25 mM Trizma base, 5 mM MgCl₂, 5 mM MnCl2, 0.05% TritonX-100 and 2 µM ATP for 45 min at RT. Reaction volume is 20 µM. Stopsolution is added for a final concentration of 100 µM EDTA. Finally 0.065 mg/ml PT66-K and 10.42 µM SA-XL665 are added in 50 mM Hepes, 0.5 M KF and 0.1% BSA. The plate is read in a Discovery instrument after 60 min incubation.

The compounds of the examples have an IC50 less than 25 µM

## Claims

1. A compound of formula (I): 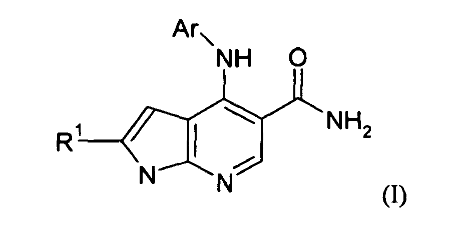 wherein:
Ar is phenyl which can be optionally substituted by one or more groups selected from halogen, hydroxy, cyano, C₁-C₈ alkyl (itself optionally substituted by one or more hydroxy or cyano groups or fluorine atoms), CH₂-R²,; CH₂O(CH₂)ₙOC₁₋₆ alkyl, C₁-C₈ alkyl-NR³-R⁴;
R² is a 5 to 7-membered saturated ring containing 1 or 2 heteroatoms selected from nitrogen, oxygen and sulphur, an aryl or 5- to 7-membered heteroaryl group containing 1 to 3 heteroatoms selected from nitrogen oxygen and suphur, each of which can optionally substituted by one or more substituents selected from hydroxyl or hydroxymethyl;
R³ is hydrogen or C₁₋₆ alkyl and R⁴ is C₁₋₆ alkyl optionally substituted by one or more groups selected from hydroxyl or phenyl,
n is 1 to 4;
R¹ is hydrogen or phenyl optionally substituted by halogen, C₁-C₈ alkoxy, C₁-C₈ thioalkyl or C₁-C₈ alkyl;
and pharmaceutically acceptable salts thereof.

2. A compound according to claim 1 in which R¹ is hydrogen or phenyl optionally substituted by halogen, in particular fluoro or bromo.

3. A compound according to claim 1 or 2 in which Ar is a phenyl optionally substituted by one or more CH₂R² groups, where R² is pyrrolidine, morpholine or imidazole each of which is optionally substituted as defined in claim 1

4. A compound according to claim 1 or 2 in which Ar is a phenyl optionally substituted by one or more CH₂R² groups where R² is pyrrolidine, morpholine or imidazole each of which is optionally substituted by hydroxyl or hydroxymethyl, or Ar is a phenyl optionally substituted by one or more CH₂NR³-R⁴groups where R³ is hydrogen or methyl and R⁴ is CH₂CH₂OH, CH₂(CH₃)CH₂OH, CH₂(phenyl)CH₂OH, CH₂CH₂(OH)phenyl, CH₂CH₂(OH)CH₂OH, or CH₂OCH₂CH₂OCH₂OH or Ar is a phenyl optionally substituted by one or more ethyl or hydroxymethyl groups.

5. A compound according to any one of claims 1 to 3-2 in which the Ar group is substituted by C₁-C₈ alkyl and C₁-C₈ alkyl substituted by a hydroxy group, more preferably hydroxymethyl.

6. A compound according to claim 1 which is: 4-(2-Ethyl-phenylamino)-2-(4-fluorophenyl)-1H-pyrrolo[2,3-*b*]pyridine-5-carboxylic acid amide
4-(2-Ethyl-3-hydroxymethyl-phenylamino)-2-(4-fluorophenyl)-1*H*-pyrrolo[2,3-*d*]pyridine-5-carboxylic acid amide
4-{2-Ethyl-3-[(2-hydroxy-ethylamino)-methyl]-phenylamino}-2-(4-fluoro-phenyl)-1*H-*pyrrolo[2,3-*b*]pyridine-5-carboxylic acid amide
4-(2-Ethyl-3-{[(2-hyroxy-ethyl)-methyl-amino]-methyl}-phenylamino)-2-(4-fluorophenyl)-1*H*-pyrrolo[2,3-*b*]pyridine-5-carboxylic acid amide
4-{2-Ethyl-3-[(2-hydroxy-1-methyl-ethylamino)-methyl]-phenylamino}-2-(4-fluorophenyl)-1*H*-pyrrolo[2,3-*b*]pyridine-5-carboxylic acid amide
4-{2-Ethyl-3-[(*S*)-(2-hydroxy-1-phenyl-ethylamino)-methyl]-phenylamino}-2-(4-fluorophenyl)-1*H*-pyrrolo[2,3-*b*]pyridine-5-carboxylic acid amide
4-{2-Ethyl-3-[(2-hydroxy-2-phenyl-ethylamino)-methyl]-phenylamino}-2-(4-fluorophenyl)-1*H*-pyrrolo[2,3-*b*]pyridine-5-carboxylic acid amide
4-(2-Ethyl-3-morpholin-4-ylmethyl-phenylamino)-2-(4-fluoro-phenyl)-1*H*-pyrrolo[2,3-*b*]pyridine-5-carboxylic acid amide
4-[2-Ethyl-3-(3-hydroxy-pyrrolidin-1-ylmethyl)-phenylamino]-2-(4-fluoro-phenyl)-1*H-*pyrrolo[2,3-*b*]pyridine-5-carboxylic acid amide
4-[2-Ethyl-3-(*(R)*-2-hydroxymethyl-pyrrolidin-1-ylmethyl)-phenylamino]-2-(4-fluorophenyl)-1*H*-pyrrolo[2,3-*b*]pyridine-5-carboxylic acid amide
4-{3-[(2,3-Dihydroxy-propylamino)-methyl]-2-ethyl-phenylamino}-2-(4-fluoro-phenyl)-1*H*-pyrrolo[2,3-*b*]pyridine-5-carboxylic acid amide
4-(2-Ethyl-3-imidazol-1-ylmethyl-phenylamino)-2-(4-fluoro-phenyl)-1*H*-pyrrolo[2,3-*b*]pyridine-5-carboxylic acid amide
4-[3-(2-Ethoxy-ethoxymethyl)-2-ethyl-phenylamino]-2-(4-fluoro-phenyl)-1*H* pyrrolo[2,3-*b*]pyridine-5-carboxylic acid amide
2-(4-Bromo-phenyl)-4-(2-ethyl-phenylamino)-1*H*-pyrrolo[2,3-*b*]pyridine-5-carboxylic acid amide
4-(2-Ethyl-phenylamino)-2-phenyl-1*H*-pyrrolo[2,3-*b*]pyridine-5-carboxylic acid amide
4-(2-Ethyl-3-hydroxymethyl-phenylamino)-2-phenyl-1*H*-pyrrolo[2,3-*b*]pyridine-5-carboxylic acid amide
2-(4-Chloro-phenyl)-4-(2-ethyl-3-hydroxymethyl-phenylamino)-1*H*-pyrrolo[2,3-*b*]pyridine-5-carboxylic acid amide
2-(4-Chloro-phenyl)-4-(2-ethyl-3-imidazol-1*H*-ylmethyl-phenylamino)-1*H*-pyrrolo[2,3-*b*]pyridine-5-carboxylic acid amide
4-(2-Ethyl-phenylamino)-1*H*-pyrrolo[2,3-*b*]pyridine-5-carboxylic acid amide
or a pharmaceutically acceptable salt thereof.

7. A compound of formula (I) as defined in any one of claims 1 to 6 for use in therapy

8. A pharmaceutical composition comprising a compound of formula (I) or a pharmaceutically acceptable salt thereof in association with a pharmaceutically acceptable carrier.

9. Use of a compound as defined in claims 1 to 6 for the manufacture of a medicament for treating a disease or condition mediated by JAK3.

10. Use according to claim 9 in which the disease or condition is asthma, host versus graft rejection/transplantation or rheumatoid arthritis.

11. A process for the preparation of a compound of formula (I) which comprises:
reaction of a compound of formula (II): 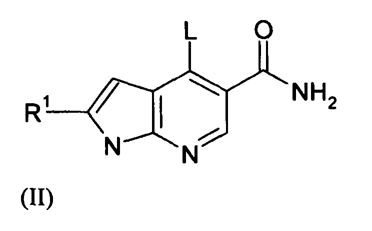
in which R¹ is as defined in formula (I) or is a protected derivatives thereof and L is a leaving group, with a compound of formula (III):
Ar-NH₂ (III)
in which Ar is as defined in formula (I) or is a protected derivatives thereof, and optionally thereafter:
• removing any protecting groups
• converting a compound of formula (I) into a further compound of formula (I)
• forming a pharmaceutically acceptable salt.

## Patentansprüche

1. Verbindung der Formel (I): 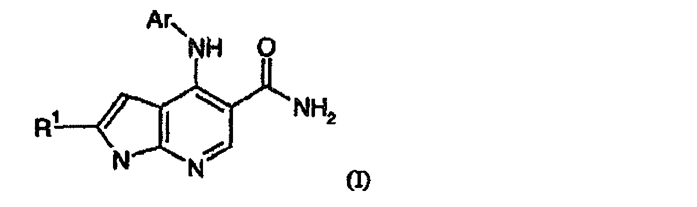 worin:
Ar Phenyl ist, welches wahlweise durch eine oder mehrere Gruppen substituiert sein kann, die aus Halogen, Hydroxy, Cyano, C₁-C₈-Alkyl (welches selbst wahlweise durch eine oder mehrere Hydroxy- oder Cyanogruppe oder Fluoratome substituiert ist), CH₂-R², CH₂O(CH₂)ₙOC₁₋₆-Alkyl, C₁-C₈-Alkyl-NR³-R⁴ gewählt wird;
R² ein 5- bis 7-gliedriger gesättigter Ring, der 1 oder 2 Heteroatome enthält, welche aus Stickstoff, Sauerstoff und Schwefel gewählt sind, eine Aryl- oder 5- bis 7-gliedrige Heteroarylgruppe, die 1 bis 3 Heteratome enthält, welche aus Stickstoff, Sauerstoff und Schwefel gewählt sind, ist, wobei jede davon wahlweise durch einen oder mehrere Substituenten substituiert sein kann, welche aus Hydroxyl oder Hydroxymethyl gewählt sind;
R³ Wasserstoff oder C₁₋₆-Alkyl ist und R⁴ C₁₋₆-Alkyl ist, wahlweise substituiert durch eine oder mehrere Gruppen, die aus Hydroxyl oder Phenyl gewählt sind,
n 1 bis 4 ist;
R¹ Wasserstoff oder Phenyl ist, wahlweise substituiert durch Halogen, C₁-C₈-Alkoxy, C₁-C₈-Thioalkyl oder C₁-C₈-Alkyl;
und pharmazeutisch annehmbare Salze davon.

2. Verbindung gemäß Anspruch 1, worin R¹ Wasserstoff oder Phenyl ist, welches wahlweise durch Halogen substituiert ist, und zwar insbesondere Fluor oder Brom.

3. Verbindung gemäß Anspruch 1 oder 2, worin Ar ein gegebenenfalls durch eine oder mehrere Gruppen CH₂R² substituiertes Phenyl ist, worin R² Pyrrolidin, Morpholin oder Imidazol ist, wobei jedes davon wahlweise so substituiert ist, wie es in Anspruch 1 definiert wurde.

4. Verbindung gemäß Anspruch 1 oder 2, worin Ar ein gegebenenfalls durch eine oder mehrere Gruppen CH₂R² substituiertes Phenyl ist, worin R² Pyrrolidin, Morpholin oder Imidazol ist, wobei jedes davon wahlweise durch Hydroxyl oder Hydroxymethyl substituiert ist, oder Ar ein gegebenenfalls durch eine oder mehrere Gruppen CH₂NR³-R⁴ substituiertes Phenyl ist, worin R³ Wasserstoff oder Methyl ist und R⁴ CH₂CH₂OH, CH₂(CH₃)CH₂OH, CH₂ Phenyl CH₂OH, CH₂CH₂(OH)Phenyl, CH₂CH₂ (OH) CH₂OH oder CH₂OCH₂CH₂OCH₂OH ist, oder Ar ein gegebenenfalls durch eine oder mehrere Ethyl- oder Hydroxymethylgruppen substituiertes Phenyl ist.

5. Verbindung gemäß mindestens einem der Ansprüche 1 bis 2, worin die Ar-Gruppe durch C₁-C₈-Alkyl und C₁-C₈-Alkyl, welches durch eine Hydroxygruppe, stärker bevorzugt Hydroxymethyl, substituiert ist, substituiert ist.

6. Verbindung gemäß Anspruch 1, welche Folgende ist:
4-(2-Ethyl-phenylamino)-2-(4-fluorphenyl)-1*H-*pyrrolo[2,3-*b*]pyridin-5-carbonsäureamid
4-(2-Ethyl-3-hydroxymethyl-phenylamino)-2-(4-fluorphenyl)-1*H*-pyrrolo[2,3-*b*]pyridin-5-carbonsäureamid
4-{2-Ethyl-3-[(2-hydroxy-ethylamino)-methyl]-phenylamino}-2-(4-fluor-phenyl)-1*H*-pyrrolo[2,3-*b*]pyridin-5-carbonsäureamid
4-(2-Ethyl-3-{[(2-hydroxy-ethyl)-methyl-amino]-methyl}-phenylamino)-2-(4-fluor-phenyl)-1*H-*pyrrolo[2,3-*b*]pyridin-5-carbonsäureamid
4-{2-Ethyl-3-[(2-hydroxy-1-methyl-ethylamino)-methyl]-phenylamino}-2-(4-fluor-phenyl)-1*H-*pyrrolo[2,3-*b*]pyridin-5-carbonsäureamid
4-{2-Ethyl-3-[(S)-(2-hydroxy-1-phenyl-ethylamino)-methyl]-phenylamino}-2-(4-fluor-phenyl)-1*H-*pyrrolo[2,3-*b*]pyridin-5-carbonsäureamid
4-{2-Ethyl-3-[(2-hydroxy-2-phenyl-ethylamino)-methyl]-phenylamino}-2-(4-fluor-phenyl)-1*H-*pyrrolo[2,3-*b*]pyrridin-5-carbonsäureamid
4-(2-Ethyl-3-morpholin-4-ylmethyl-phenylamino)-2-(4-fluor-phenyl)-1*H*-pyrrolo[2,3-*b*]pyridin-5-carbonsäureamid
4-[2-Ethyl-3-(3-hydroxy-pyrrolidin-1-ylmethyl)-phenylamino]-2-(4-fluor-phenyl)-1*H*-pyrrolo[2,3-*b*]pyridin-5-carbonsäureamid
4-(2-Ethyl-3-((*R*)-2-hydroxymethyl-pyrrolidin-1-ylmethyl)-phenylamino]-2-(4-fluor-phenyl)-1*H-*pyrrolo[2,3-*b*]pyridin-5-carbonsäureamid
4-{3-[(2,3-Dihydroxy-propylamino)-methyl]-2-ethyl-phenylamino}-2-(4-fluor-phenyl)-1*H*-pyrrolo[2,3-*b*]pyridin-5-carbonsäureamid
4-(2-Ethyl-3-imidazol-1-ylmethyl-phenylamino)-2-(4-fluor-phenyl)-1*H*-pyrrolo[2,3-*b*]pyridin-5-carbonsäureamid
4-[3-(2-Ethoxy-ethoxymethyl)-2-ethyl-phenylamino]-2-(4-fluor-phenyl)-1*H*-pyrrolo[2,3-*b*]pyridin-5-carbonsäureamid
2-(4-Brom-phenyl)-4-(2-ethyl-phenylamino)-1*H-*pyrrolo[2,3-*b*]pyridin-5-carbonsäureamid
4-(2-Ethyl-phenylamino)-2-phenyl-1*H*-pyrrolo[2,3-*b*]pyridin-5-carbonsäureamid
4-(2-Ethyl-3-hydroxymethyl-phenylamino)-2-phenyl-1*H*-pyrrolo[2,3-*b*]pyridin-5-carbonsäureamid
2-(4-Chlor-phenyl)-4-(2-ethyl-3-hydroxymethyl-phenylamino)-1*H*-pyrrolo[2,3-*b*]pyridin-5-carbonsäureamid
2-(4-Chlor-phenyl)-4-(2-ethyl-3-imidazol-1-ylmethyl-phenylamino)-1*H*-pyrrolo[2,3-*b*]pyridin-5-carbonsäureamid
4-(2-Ethyl-phenylamino)-1*H*-pyrrolo[2,3-*b*]pyridin-5-carbonsäureamid
oder ein pharmazeutisch annehmbares Salz davon.

7. Verbindung der Formel (I) wie in mindestens einem der Ansprüche 1 bis 6 definiert, zur Verwendung in der Therapie.

8. Pharmazeutische Zusammensetzung, umfassend eine Verbindung der Formel (I) oder ein pharmazeutisches annehmbares Salz davon in Assoziation mit einem pharmazeutisch annehmbaren Träger.

9. Verwendung einer Verbindung, wie in den Ansprüchen 1 bis 6 definiert, zur Herstellung eines Medikaments zur Behandlung einer Erkrankung oder eines Zustandes, der durch JAK3 vermittelt wird.

10. Verwendung gemäß Anspruch 9, wobei die Erkrankung oder der Zustand Asthma, Wirt-anti-Transplantat-Abstoßung/Transplantation oder rheumatoide Arthritis ist.

11. Verfahren zur Herstellung einer Verbindung der Formel (I), welches Folgendes umfasst:
Umsetzung einer Verbindung der Formel (II): 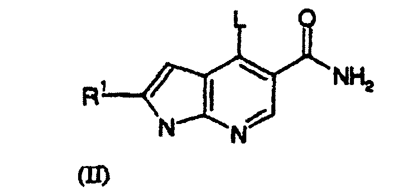
worin R¹ wie in der Formel (I) definiert ist oder ein geschütztes Derivat davon ist und L eine Abgangsgruppe ist, mit einer Verbindung der Formel (III):
Ar-NH₂ (III)
worin Ar in der Formel (I) definiert ist oder ein geschütztes Derivat davon ist, und gegebenenfalls danach:
• Entfernen jedweder Schutzgruppen
• Umwandeln einer Verbindung der Formel (I) in eine weitere Verbindung der Formel (I)
• Bilden eines pharmazeutisch annehmbaren Salzes.

## Revendications

1. Composé de formule (I) : 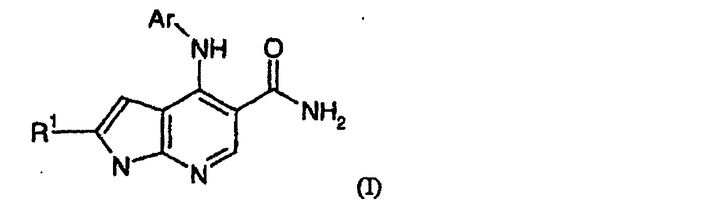 dans laquelle:
Ar est un groupe phényle qui peut être éventuellement substitué par un ou plusieurs groupes choisis parmi les groupes halogéno, hydroxy, cyano, alkyle en C₁-C₈ (lui-même éventuellement substitué par un ou plusieurs groupes hydroxy ou cyano ou atomes de fluor), CH₂-R², CH₂O(CH₂)ₙO-C₁₆-alkyle-C, C₁-C₈-alkyl -NR³-R⁴ ;
R² est un noyau saturé à 5 à 7 chaînons contenant 1 ou 2 hétéroatomes choisis parmi l'azote, l'oxygène et le soufre, un groupe aryle ou un groupe hétéroaryle à 5 à 7 chaînons contenant 1 à 3 hétéroatomes choisis parmi l'azote, l'oxygène et le soufre, chacun d'eux pouvant être éventuellement substitué par un ou plusieurs substituants choisis parmi les groupes hydroxyle ou hydroxyméthyle ;
R³ est un atome d'hydrogène ou un groupe alkyle en C₁-C₆ et R⁴ est un groupe alkyle en C₁-C₆ éventuellement substitué par un ou plusieurs groupes choisis parmi les groupes hydroxyle ou phényle
n a une valeur de 1 à 4 ;
R¹ est un atome d'hydrogène ou un groupe phényle éventuellement substitué par un groupe halogéno, alcoxy en C₁-C₈, thioalkyle en C₁-C₈ ou alkyle en C₁-C₈;
et sels pharmaceutiquement acceptables de ce composé.

2. Composé selon la revendication 1 dans lequel R¹ est un atome d'hydrogène ou un groupe phényle éventuellement substitué par un groupe halogéno, en particulier fluoro ou bromo.

3. Composé selon la revendication 1 ou 2 dans lequel Ar est un groupe phényle éventuellement substitué par un ou plusieurs groupes CH₂R² dans lesquels R² est un radical pyrrolidine, morpholine ou imidazole, chacun d'eux étant éventuellement substitué de la manière définie dans la revendication 1.

4. Composé selon la revendication 1 ou 2 dans lequel Ar est un groupe phényle éventuellement substitué par un ou plusiers groupes CH₂R² dans lesquels R² est un radical pyrrolidine, morpholine ou imidazole, chacun d'eux étant éventuellement substitué par un groupe hydroxyle ou hydroxyméthyle, ou Ar est un groupe phényle éventuellement substitué par un ou plusieurs groupes CH₂NR³-R⁴ dans lesquels R³ est un atome d'hydrogène ou un groupe méthyle et R⁴ est CH₂CH₂OH, CH₂(CH₃)CH₂OH, CH₂(phényl)CH₂OH, CH₂CH₂ (OH) phényle, CH₂CH₂(OH)CH₂OH ou CH₂OCH₂CH₂OCH₂OH, ou Ar est un groupe phényle éventuellement substitué par un ou plusieurs groupes éthyle ou hydroxyméthyle.

5. Composé selon l'une quelconque des revendications 1 à 2 dans lequel le groupe Ar est substitué par un groupe alkyle en C₁-C₈ et alkyle en C₁-C₈ substitué par un groupe hydroxy, mieux encore hydroxyméthyle.

6. Composé selon la revendication 1 qui est :
l'amide d'acide 4-(2-éthylphénylamino)-2-(4-fluorophényl)-1H-pyrrolo[2,3-b]pyridine-5-carboxylique
l'amide d'acide 4-(2-éthyl-3-hydroxyméthylphénylamino)-2-(4-fluorophényl)-1H-pyrrolo[2,3-b]pyridine-5-carboxylique
l'amide d'acide 4-{2-éthyl-3-[(2-hydroxyéthylamino)-méthyl]phénylamino}-2-(4-fluorophényl)-1H-pyrrolo-[2,3-b]pyridine-5-carboxylique
l'amide d'acide 4-(2-éthyl-3-{[(2-hydroxyéthyl)méthylamino]méthyl}phénylamino)-2-(4-fluorophényl)-1H-pyrrolo[2,3-b]pyridine-5-carboxylique
l'amide d'acide 4-{2-éthyl-3-[(2-hydroxy-1-méthyléthylamino)méthyl]phénylamino}-2-(4-fluorophényl)-1H-pyrrolo[2,3-b]pyridine-5-carboxylique
l'amide d'acide 4-{2-éthyl-3-[(S)-(2-hydroxy-1-phényléthylamino)méthyl]phénylamino}-2-(4-fluorophényl)-1H-pyrrolo[2,3-b]pyridine-5-carboxylique
l'amide d'acide 4-{2-éthyl-3-[(2-hydroxy-2-phényléthylamino)méthyl]phénylamino}-2-(4-fluorophényl)-1H-pyrrolo[2,3-b]pyridine-5-carboxylique
l'amide d'acide 4-(2-éthyl-3-morpholin-4-ylméthylphénylamino)-2-(4-fluorophényl)-1H-pyrrolo[2,3-b]-pyridine-5-carboxylique
l'amide d'acide 4-[2-éthyl-3-(3-hydroxypyrrolidin-1-yl-méthyl)phénylamino]-2-(4-fluorophényl)-1H-pyrrolo-[2,3-b]pyridine-5-carboxylique
l'amide d'acide 4-[2-éthyl-3-((R)-2-hydroxyméthylpyrrolidin-1-ylméthyl)phénylamino]-2-(4-fluorophényl)-1H-pyrrolo[2,3-b]pyridine-5-carboxylique
l'amide d'acide 4-{3-[(2,3-dihydroxypropylamino)-méthyl]-2-éthylphénylamino}-2-(4-fluorophényl)-1H-pyrrolo[2,3-b]pyridine-5-carboxylique
l'amide d'acide 4-(2-éthyl-3-imidazol-1-ylméthylphénylamino)-2-(4-fluorophényl)-1H-pyrrolo[2,3-b]-pyridine-5-carboxylique
l'amide d'acide 4-[3-(2-éthoxyéthoxyméthyl)-2-éthylphénylamino]-2-(4-fluorophényl)-1H-pyrrolo[2,3-b]-pyridine-5-carboxylique
l'amide d'acide 2-(4-bromophényl)-4-(2-éthylphénylamino)-1H-pyrrolo[2,3-b]pyridine-5-carboxylique
l'amide d'acide 4-(2-éthylphénylamino)-2-phényl-1H-pyrrolo[2,3-b]pyridine-5-carboxylique
l'amide d'acide 4-(2-éthyl-3-hydroxyméthylphénylamino)-2-phényl-1H-pyrrolo[2,3-b]pyridine-5-carboxylique
l'amide d'acide 2-(4-chlorophényl)-4-(2-éthyl-3-hydroxyméthylphénylamino)-1H-pyrrolo[2,3-b]pyridine-5-carboxylique
l'amide d'acide 2-(4-chlorophényl)-4-(2-éthyl-3-imidazol-1-ylméthylphénylamino)-1H-pyrrolo[2,3-b]-pyridine-5-carboxylique
l'amide d'acide 4-(2-éthylphénylamino)-1H-pyrrolo-[2,3-b]pyridine-5-carboxylique
ou sel pharmaceutiquement acceptable de ce composé.

7. Composé de formule (I) tel que défini dans l'une quelconque des revendications 1 à 6 à utiliser en thérapie.

8. Composition pharmaceutique comprenant un composé de formule (I) ou un sel pharmaceutiquement acceptable de ce composé en association avec un véhicule pharmaceutiquement acceptable.

9. Utilisation d'un composé tel que défini dans les revendications 1 à 6 pour la fabrication d'un médicament destiné à traiter une maladie ou une affection médiée par JAK3.

10. Utilisation selon la revendication 9 dans laquelle la maladie ou l'affection est l'asthme, une transplantation réaction de rejet hôte contre greffe ou la polyarthrite rhumatoïde.

11. Procédé de préparation d'un composé de formule (I) qui comprend les étapes consistant à :
faire réagir un composé de formule (II) : 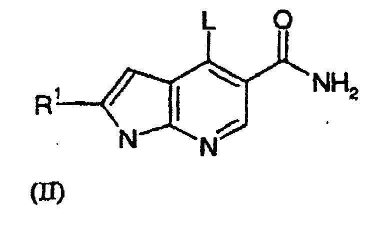
dans laquelle R¹ est tel que défini dans la formule (I) ou est un de ses dérivés protégés et L est un groupe partant, avec un composé de formule (III):
Ar-NH₂ (III)
dans laquelle Ar est tel que défini dans la formule (I) ou est un de ses dérivés protégés,
et éventuellement ensuite :
• éliminer les éventuels groupes protecteurs,
• transformer un composé de formule (I) en un autre composé de formule (I)
• former un sel pharmaceutiquement acceptable.
